# EUROPEAN PATENT APPLICATION

(11) **EP 4 559 405 A1**
(43) Date of publication of application: **28.05.2025**
(21) Application number: 24167163.5
(22) Date of filing: 28.03.2024
(51) Int. Cl.: A61B 8/00, A61B 8/08

(54) **ULTRASOUND IMAGING APPARATUS AND METHOD FOR PROVIDING USER INTERFACE**

(30) Priority: 22.11.2023 KR 20230163783
(71) Applicant: Samsung Medison Co., Ltd., Hongcheon-gun, Gangwon-do 25108 (KR)
(72) Inventor: Yu, Hwanseung, 05340 Seoul (KR); Na, Minsoo, 05340 Seoul (KR)
(74) Representative: Frey, Sven Holger

(57) **Abstract**

An ultrasound imaging apparatus for providing a user interface (UI) for an ultrasound image, and a control method of the ultrasound imaging apparatus are provided. The ultrasound imaging apparatus may include a display, a motion sensor, an input interface, and at least one processor configured to display, via the display, a UI image, corresponding to a measurement parameter, on the ultrasound image, change, via the motion sensor, a position of the UI image within the ultrasound image according to movement of the ultrasound imaging apparatus, determine, in response to receiving a user input for setting the measurement parameter via the input interface, a value of the measurement parameter based on the position of the UI image within the ultrasound image, and display a measurement value for an object based on the determined value of the measurement parameter.

## Description

### CROSS-REFERENCE TO RELATED APPLICATION

This application is based on and claims priority under 35 U.S.C. §119 to Korean Patent Application No. 10-2023-0163783, filed on November 22, 2023, in the Korean Intellectual Property Office, the disclosure of which is incorporated by reference herein in its entirety.

### BACKGROUND

### 1. Field

The disclosure relates to an ultrasound imaging apparatus providing a user interface based on movement of the ultrasound imaging apparatus, a control method of the ultrasound imaging apparatus, and a computer-readable recording medium having stored therein a computer program for performing the control method of the ultrasound imaging apparatus.

### 2. Description of the Related Art

Recently, in the medical field, various types of medical imaging apparatuses have been widely used to visualize and obtain information about living tissue of a human body for early diagnosis or surgery with regard to various diseases. Representative examples of these medical imaging apparatuses may include an ultrasound imaging apparatus, a computed tomography (CT) apparatus, and a magnetic resonance imaging (MRI) apparatus.

Ultrasound imaging apparatuses transmit ultrasound signals generated by transducer elements of a probe to an object and receive information of signals reflected from the object, thereby obtaining at least one image of an internal part (e.g., soft tissue or blood flow) of the object. In particular, ultrasound imaging apparatuses are used for medical purposes including observing an internal area of an object, detecting foreign substances, and assessing injuries. Such ultrasound imaging apparatuses exhibit high stability, are capable of displaying images in real time, and are safe due to lack of radiation exposure, as compared to X-ray apparatuses, and therefore, have been widely used together with other types of imaging diagnostic apparatuses.

### SUMMARY

Additional aspects will be set forth in part in the description which follows and, in part, will be apparent from the description, or may be learned by practice of the presented embodiments of the disclosure.

According to an aspect of the disclosure, an ultrasound imaging apparatus may be provided. The ultrasound imaging apparatus may include a display, a motion sensor, an input interface, and at least one processor configured to display, via the display, a user interface (Ul) image, corresponding to a measurement parameter, on an ultrasound image, change, via the motion sensor, a position of the UI image within the ultrasound image according to movement of the ultrasound imaging apparatus, determine, in response to receiving a user input for setting the measurement parameter via the input interface, a value of the measurement parameter based on the position of the UI image within the ultrasound image, and display a measurement value for an object based on the determined value of the measurement parameter.

According to an aspect of the disclosure, a control method of an ultrasound imaging apparatus may be provided. The control method of the ultrasound imaging apparatus may include displaying a UI image, corresponding to a measurement parameter, on an ultrasound image, changing a position of the UI image within the ultrasound image according to movement of the ultrasound imaging apparatus, determining, in response to receiving a user input for setting the measurement parameter, a value of the measurement parameter based on the position of the UI image within the ultrasound image; and displaying a measurement value for an object based on the determined value of the measurement parameter.

According to an aspect of the disclosure, there may be provided a computer-readable recording medium having recorded thereon a program for performing, on a computer, a control method of an ultrasound imaging apparatus.

### BRIEF DESCRIPTION OF THE DRAWINGS

The above and other aspects, features, and advantages of certain embodiments of the disclosure will be more apparent from the following description taken in conjunction with the accompanying drawings, in which:
FIG. 1 illustrates a method, performed by an ultrasound imaging apparatus, of providing a user interface based on movement of the ultrasound imaging apparatus by a user, according to an embodiment;
FIG. 2 is a block diagram of an ultrasound imaging apparatus according to an embodiment;
FIG. 3 is a flowchart of a method, performed by an ultrasound imaging apparatus, of providing a user interface based on movement of the ultrasound imaging apparatus by a user, according to an embodiment;
FIG. 4 is a flowchart of a method, performed by an ultrasound imaging apparatus, of setting Doppler parameters in a spectral Doppler measurement mode, according to an embodiment;
FIG. 5 illustrates a method, performed by an ultrasound imaging apparatus, of setting a parameter, according to an embodiment;
FIG. 6 illustrates a method, performed by an ultrasound imaging apparatus, of setting parameters, according to an embodiment;
FIG. 7 illustrates a method, performed by an ultrasound imaging apparatus, of setting parameters, according to an embodiment;
FIG. 8 illustrates a method, performed by an ultrasound imaging apparatus, of setting parameters, according to an embodiment;
FIGS. 9A and 9B illustrate a method, performed by an ultrasound imaging apparatus, of setting parameters on an ultrasound image by using a linear probe, according to an embodiment;
FIG. 10 is a flowchart of a method, performed by an ultrasound imaging apparatus, of enlarging a region of an ultrasound image as it enters a measurement mode, according to an embodiment;
FIGS. 11A and 11B illustrate a method, performed by an ultrasound imaging apparatus, of providing a user interface for distance measurement, according to an embodiment;
FIG. 12 illustrates a method, performed by an ultrasound imaging apparatus, of setting a measurement parameter, according to an embodiment;
FIG. 13 illustrates a method, performed by an ultrasound imaging apparatus, of setting Doppler parameters, according to an embodiment;
FIGS. 14A and 14B are block diagrams of configurations of an ultrasound imaging system according to an embodiment; and
FIGS. 15A and 15B are diagrams illustrating examples of an ultrasound imaging apparatus according to an embodiment.

### DETAILED DESCRIPTION

Throughout the disclosure, the expression "at least one of a, b or c" indicates only a, only b, only c, both a and b, both a and c, both b and c, all of a, b, and c, or variations thereof.

Embodiments will be described more fully hereinafter with reference to the accompanying drawings so that they may be easily implemented by one of ordinary skill in the art to which the disclosure belongs. However, the disclosure may be implemented in different forms and should not be construed as being limited to the embodiments set forth herein. Furthermore, parts not related to descriptions of the disclosure are omitted to clearly explain the disclosure in the drawings, and like reference numerals denote like elements throughout.

As the terms used herein, general terms that are currently widely used are selected by taking functions in the disclosure into account, but the terms are intended to encompass various other terms depending on an intention of those skilled in the art, precedent cases, advent of new technologies, etc. Thus, the terms used herein should be defined not by simple appellations thereof but based on the meaning of the terms together with the overall description of the disclosure.

Furthermore, although the terms including an ordinal number such as "first", "second", etc. may be used herein to describe various elements or components, these elements or components should not be limited by the terms. The terms are only used to distinguish one element or component from another element or component.

In addition, the terms used herein are only used to describe particular embodiments, and are not intended to limit the disclosure. Singular expressions used herein are intended to include plural expressions as well unless the context clearly indicates otherwise. Furthermore, throughout the specification, it will be understood that when a part is referred to as being "connected" or "coupled" to another part, it may be directly connected to or electrically coupled to the other part with one or more intervening elements therebetween. Furthermore, throughout the specification, when a part "includes" or "comprises" an element, unless there is a particular description contrary thereto, it is understood that the part may further include other elements, not excluding the other elements.

Expressions such as "in some embodiments of the disclosure" or "in an embodiment of the disclosure" described in various parts of this specification do not necessarily refer to the same embodiment(s).

Also, as used herein, an 'object' is a target to be imaged, and may include a human, an animal, or a part thereof. For example, the object may include a part of a body (organ, tissue, or the like), or a phantom.

Throughout the specification, an 'ultrasound image' refers to an image of an object generated or processed based on ultrasound signals transmitted to the object and reflected therefrom.

Embodiments provide an ultrasound imaging apparatus for providing a user interface based on movement of the ultrasound imaging apparatus, and a control method of the ultrasound imaging apparatus.

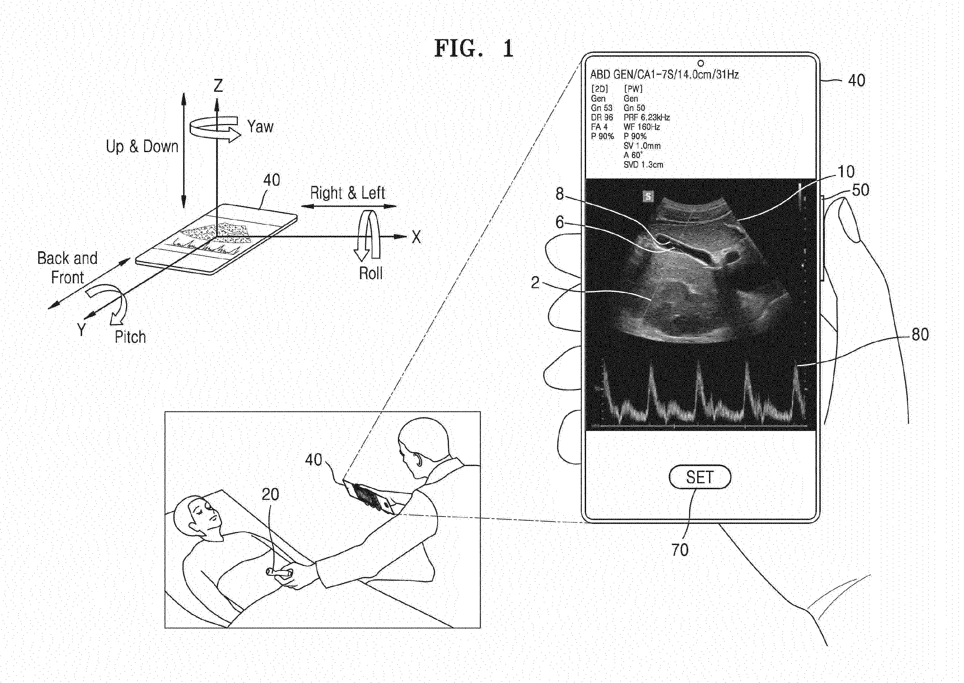

FIG. 1 illustrates a method, performed by an ultrasound imaging apparatus 40, of providing a user interface (Ul) based on movement of the ultrasound imaging apparatus 40 by a user, according to an embodiment.

Referring to FIG. 1, the ultrasound imaging apparatus 40 may change a position of a UI image corresponding to a measurement parameter according to movement of the ultrasound imaging apparatus 40. A user may change a value of a measurement parameter by changing at least one of a position or an angle of the ultrasound imaging apparatus 40.

A measurement parameter may be a measurement point or parameter that is set on an ultrasound image of an object to measure the object. For example, when a measurement item is a length of a region of the object, a measurement parameter may be locations of two points on the ultrasound image. Furthermore, when a measurement item is a spectral Doppler waveform of the object, measurement parameters may be Doppler parameters including one axial line (Doppler line), a position of a sample volume, a size of the sample volume, and a Doppler angle on an ultrasound image.

The ultrasound imaging apparatus 40 may display a user interface (Ul) image representing a measurement parameter on an ultrasound image. For example, as shown in a diagram on the right of FIG. 1, in a spectral Doppler measurement mode, the ultrasound imaging apparatus 40 may display UI images representing measurement parameters on a brightness mode (B-mode) image (hereinafter referred to as an ultrasound image) 10. In FIG. 1, the UI images representing the measurement parameters may include a line image 2 corresponding to a Doppler line, a sample volume image 6 corresponding to a sample volume, and an angle image 8 corresponding to a Doppler angle. The angle image 8 may be referred to as a blood flow direction image indicating a direction of blood flow.

The ultrasound imaging apparatus 40 may receive, via a motion sensor (not shown), a user input for changing at least one of a position or an angle of the ultrasound imaging apparatus 40. According to an embodiment, as shown in a diagram on the left of FIG. 1, the motion sensor provided in the ultrasound imaging apparatus 40 may detect a distance of movement of the ultrasound imaging apparatus 40 in a direction of an X-axis, a distance of movement in a direction of a Y-axis, a distance of movement in a direction of a Z-axis, a roll value representing an angle of rotation about the X axis, a pitch value representing an angle of rotation about the Y axis, and a yaw value representing an angle of rotation about the Z axis.

In response to the user changing at least one of a position or an angle of the ultrasound imaging apparatus 40, the ultrasound imaging apparatus 40 may detect, via the motion sensor, displacements in the X-axis direction, the Y-axis direction, and the Z-axis direction and changes in a roll value, a pitch value, and a yaw value.

The ultrasound imaging apparatus 40 may change positions of the UI images, i.e., the line image 2, the sample volume image 6, and the angle image 8, within the ultrasound image 10, based on a change in at least one of the position or the angle of the ultrasound imaging apparatus 40.

For example, in response to receiving a user input for moving the ultrasound imaging apparatus 40 to the right (in the X-axis direction), the ultrasound imaging apparatus 40 may move the ultrasound image 10 in a right lateral direction, thereby moving the line image 2 corresponding to the Doppler line in a left lateral direction relative to the ultrasound image 10.

Furthermore, for example, in response to receiving a user input for moving the ultrasound imaging apparatus 40 upward (in the Z-axis direction), the ultrasound imaging apparatus 40 may move the ultrasound image 10 upward along an axial direction, thereby moving the sample volume image 6 corresponding to the sample volume downward along the axis direction relative to the ultrasound image 10.

Furthermore, for example, in response to receiving a user input for moving the ultrasound imaging apparatus 40 forward (in the Y-axis direction), the ultrasound imaging apparatus 40 may significantly change a size of the sample volume image 6 within the ultrasound image 10.

Furthermore, for example, in response to receiving a user input for rotating the ultrasound imaging apparatus 40 clockwise about the Z-axis, the ultrasound imaging apparatus 40 may rotate the angle image 8 clockwise within the ultrasound image 10.

In addition, for example, in response to receiving a user input for moving the ultrasound imaging apparatus 40 forward, backward, left, or right in the X-Y plane, the ultrasound imaging apparatus 40 may change a location of a measurement point image within the ultrasound image 10 by moving the ultrasound image 10 forward, backward, left, or right.

Based on receiving a user input for setting a measurement parameter, the ultrasound imaging apparatus 40 may determine at least one of a position or an angle of a UI image within the ultrasound image 10 as a value of the measurement parameter.

The user input for setting a measurement parameter may include, for example, a user input for selecting a software button 70 displayed on a screen of the ultrasound imaging apparatus 40. Furthermore, the user input for setting a measurement parameter may include a user input for selecting a hardware button 50 provided on a side of the ultrasound imaging apparatus 40. In addition, the user input for setting a measurement parameter may include a user input for releasing the hardware button 50 provided on the side of the ultrasound imaging apparatus 40.

Referring to the right diagram of FIG. 1, the ultrasound imaging apparatus 40 may determine a position of the line image 2 within the ultrasound image 10 as a Doppler line, determine a position and a size of the sample volume image 6 therein as a position and a size of the sample volume, and determine an angle of the angle image 8 therein as a Doppler angle.

The ultrasound imaging apparatus 40 may obtain measurement values based on determined values of measurement parameters. For example, the ultrasound imaging apparatus 40 may obtain a spectral Doppler waveform generated based on the determined Doppler angle and the determined position and size of the sample volume.

The ultrasound imaging apparatus 40 may be a portable apparatus as shown in FIG. 1. In this case, while holding the probe 20 in contact with an object with one hand, the user needs to operate the ultrasound imaging apparatus 40 with only the other hand. Furthermore, due to a small size of a display provided in the ultrasound imaging apparatus 40, when the user selects a measurement point with his or her finger, the measurement point may be obscured by the finger.

The user may minimize touches on the display by changing the position or angle of the ultrasound imaging apparatus 40 to set measurement parameters while holding the ultrasound imaging apparatus 40 with his or her hand. Accordingly, the measurement point may not be obscured by the user's finger, or the display may not be contaminated by the user's fingerprint. The user may also set parameters or measurement points accurately with just one hand.

According to an embodiment, based on receiving a user input for entering a measurement mode, the ultrasound imaging apparatus 40 may enlarge a region of the displayed ultrasound image 10 and display a UI image on the enlarged region.

By automatically enlarging the ultrasound image 10 when entering the measurement mode, a region to be measured may be shown in detail on a small screen without a separate user input, and thus, the user may set a more accurate point.

According to an embodiment, measurement parameters set by the ultrasound imaging apparatus 40 may include a plurality of measurement parameters having a predetermined setting order. For example, when a measurement item is a spectral Doppler waveform of the object, measurement parameters may be set in the order of a Doppler line, a position of a sample volume, a size of the sample volume, and a Doppler angle.

When receiving a user input for setting a first measurement parameter from among the plurality of measurement parameters, the ultrasound imaging apparatus 40 may display a UI image corresponding to a second measurement parameter following the first measurement parameter. For example, when receiving a user input for setting the Doppler line, the ultrasound imaging apparatus 40 may display a UI image for setting the position of the sample volume.

In the case of setting measurement parameters with a predetermined setting order, when one measurement parameter is set, a UI image corresponding to a next measurement parameter to be set is automatically displayed, so that the user may not need to separately perform a separate input for setting the next measurement parameter.

According to an embodiment, the ultrasound imaging apparatus 40 may change a position of a UI image within the ultrasound image 10 in response to receiving a user input for changing at least one of a position or an angle of the ultrasound imaging apparatus 40 while a predetermined hardware button 50 is pressed.

Furthermore, in response to receiving a user input for releasing the predetermined hardware button that has been pressed, the ultrasound imaging apparatus 40 may determine the position or angle of the UI image within the ultrasound image 10 as a value of a measurement parameter.

Because it is possible to receive a user input for starting, proceeding with, and ending settings for measurement parameters by using only one hardware button, measurement parameters may be set without touching the display, and shaking of the ultrasound imaging apparatus 40 caused by touching the display may be minimized.

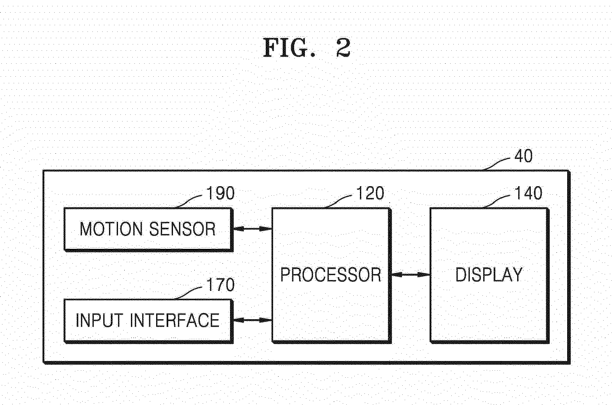

FIG. 2 is a block diagram of the ultrasound imaging apparatus 40 according to an embodiment.

Referring to FIG. 2, the ultrasound imaging apparatus 40 may include a processor 120, a display 140, an input interface 170, and a motion sensor 190.

According to an embodiment, the ultrasound imaging apparatus 40 may include a portable ultrasound imaging apparatus.

The processor 120 may control all operations of the ultrasound imaging apparatus 40 and operations of components of the ultrasound imaging apparatus 40.
The processor 120 may include at least one processor. The processor 120 may also control an operation of the ultrasound imaging apparatus 40 by receiving a control signal from the input interface 170 or an external device.

The display 140 may display an ultrasound image and various pieces of information processed by the ultrasound imaging apparatus 40 or the probe 20. Furthermore, the display 140 may include a touch panel or a touch screen.

The input interface 170 may receive a user input for controlling the ultrasound imaging apparatus 40. For example, the user input may include, but is not limited to, inputs for manipulating hardware buttons, keypads, mice, trackballs, jog switches, or knops, an input for touching a touchpad or a touch screen, a voice input, a motion input, and an input of biometric information.

The motion sensor 190 may detect a movement direction, a movement distance, and a rotation angle of the ultrasound imaging apparatus 40. The motion sensor 190 may include, but is not limited to, an acceleration sensor and a gyroscope.

The processor 120 may display, on the display 140, a UI image representing a measurement parameter on an ultrasound image.

The processor 120 may receive, via the motion sensor 190, a user input for changing at least one of a position or an angle of the ultrasound imaging apparatus 40.

The processor 120 may change a position of the UI image within the ultrasound image according to at least one of the position or the angle of the ultrasound imaging apparatus 40. The processor 120 may receive, via the input interface 170, a user input for setting a measurement parameter. Furthermore, in response to receiving the user input for setting the measurement parameter, the processor 120 may determine a value of the measurement parameter based on a position of the UI image within the ultrasound image. The processor 120 may display a measurement value for an object based on the determined value of the measurement parameter.

The processor 120 may move the ultrasound image in a lateral direction in response to receiving a user input for changing at least one of the position or the angle of the ultrasound imaging apparatus 40.

In response to receiving a user input for changing at least one of the position or the angle of the ultrasound imaging apparatus 40, the processor 120 moves the ultrasound image in an axial direction, thereby moving the UI image within the ultrasound image in the axial direction.

The processor 120 may change a size of the UI image within the ultrasound image in response to receiving a user input for changing at least one of the position or the angle of the ultrasound imaging apparatus 40.

The processor 120 may change an angle of the UI image in the ultrasound image in response to receiving a user input for changing at least one of the position or the angle of the ultrasound imaging apparatus 40.

In response to receiving a user input for changing at least one of the position or
the angle of the ultrasound imaging apparatus 40,
the processor 120 may move the ultrasound image up, down, left, or right, thereby changing a position of the UI image within the ultrasound image up, down, left, or right.

In response to receiving a user input for setting a first measurement parameter from among a plurality of measurement parameters, the processor 120 may display, on the display 140, a UI image corresponding to a second measurement parameter following the first measurement parameter.

The processor 120 may display an ultrasound image on the display 140.

Based on receiving a user input for entering a measurement mode, the processor 120 may enlarge a region of the displayed ultrasound image, and display a UI image on the enlarged region.

The processor 120 may display, on the display 140, guidance information that guides a direction of movement of the ultrasound imaging apparatus 40 for moving a UI image within the ultrasound image.

When a predetermined hardware button is pressed, the processor 120 may receive, via the motion sensor 190, a user input for changing at least one of the position or the angle of the ultrasound imaging apparatus 40.

The processor 120 may determine whether the predetermined hardware button has been pressed via the input interface 170.

The processor 120 may receive a user input for releasing the pressed hardware button via the input interface 170. In addition, in response to receiving the user input for releasing the pressed hardware button via the input interface 170, the processor 120 may determine a position of a UI image within the ultrasound image as a value of a measurement parameter.

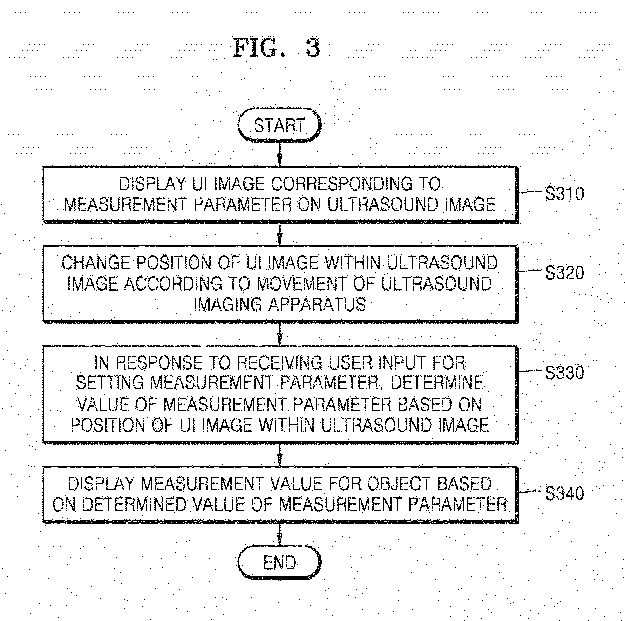

FIG. 3 is a flowchart of a method, performed by the ultrasound imaging apparatus 40, of providing a UI based on movement of the ultrasound imaging apparatus 40 by the user, according to an embodiment.

In operation S310, the ultrasound imaging apparatus 40 may display a UI image, corresponding to a measurement parameter, on an ultrasound image.

Based on receiving a user input for selecting a measurement item, the ultrasound imaging apparatus 40 may display a UI image representing a measurement parameter corresponding to a measurement item on the ultrasound image.

For example, the ultrasound imaging apparatus 40 may display a UI image indicating a measurement point based on receiving a user input for selecting a length measurement item.

In operation S320, the ultrasound imaging apparatus 40 may change a position of the UI image within the ultrasound image according to movement of the ultrasound imaging apparatus 40.

The ultrasound imaging apparatus 40 may receive a user input for moving the ultrasound imaging apparatus 40 via a motion sensor. For example, the ultrasound imaging apparatus 40 may receive a user input for changing at least one of a position or an angle of the ultrasound imaging apparatus 40.

The ultrasound imaging apparatus 40 may change a position of the UI image or a position of the ultrasound image, based on a change in at least one of the position or the angle of the ultrasound imaging apparatus 40.

For example, the ultrasound imaging apparatus 40 may move the ultrasound image or UI image in a direction of movement of the ultrasound imaging apparatus 40 in proportion to a distance of movement of the ultrasound imaging apparatus 40.

Furthermore, for example, the ultrasound imaging apparatus 40 may move the ultrasound image or UI image in proportion to an angle of movement of the ultrasound imaging apparatus 40.

In operation S330, in response to receiving A user input for setting a measurement parameter, the ultrasound imaging apparatus 40 may determine a value of the measurement parameter based on a position of the UI image within the ultrasound image.

In response to receiving a user input for setting a measurement parameter, the ultrasound imaging apparatus 40 may determine a value of the measurement parameter based on the position of the UI image within the ultrasound image. For example, when a measurement item is a length measurement, and a measurement parameter is a measurement point, the ultrasound imaging apparatus 40 may determine a position of a measurement point image within the ultrasound image as the measurement point. Also, for example, when a measurement item is a spectral Doppler waveform, and a measurement parameter is a Doppler line, the ultrasound imaging apparatus 40 may determine a scan line corresponding to a position of a line image as the Doppler line.

In operation S340, the ultrasound imaging apparatus 40 may display a measurement value for an object based on the determined value of the measurement parameter.

For example, when a measurement item is a length measurement, a distance between two measurement points may be displayed as a measurement value.

In addition, for example, when a measurement item is a spectral Doppler waveform, the spectral Doppler waveform may be displayed as a measurement value based on values of set Doppler parameters.

According to an embodiment, the ultrasound imaging apparatus 40 may transmit an ultrasound signal to the object based on the value of the measurement parameter, obtain a measurement value for the object based on an ultrasound signal reflected from the object, and display the obtained measurement value.

According to an embodiment, the ultrasound imaging apparatus 40 may transmit information about the value of the measurement parameter to another device. The other device may transmit an ultrasound signal to the object based on the transmitted information about the value of the measurement parameter, and receive an ultrasound signal reflected from the object. The other device may calculate a measurement value for the object based on the received ultrasound signal. The ultrasound imaging apparatus 40 may receive the measurement value from the other device and display the received measurement value.

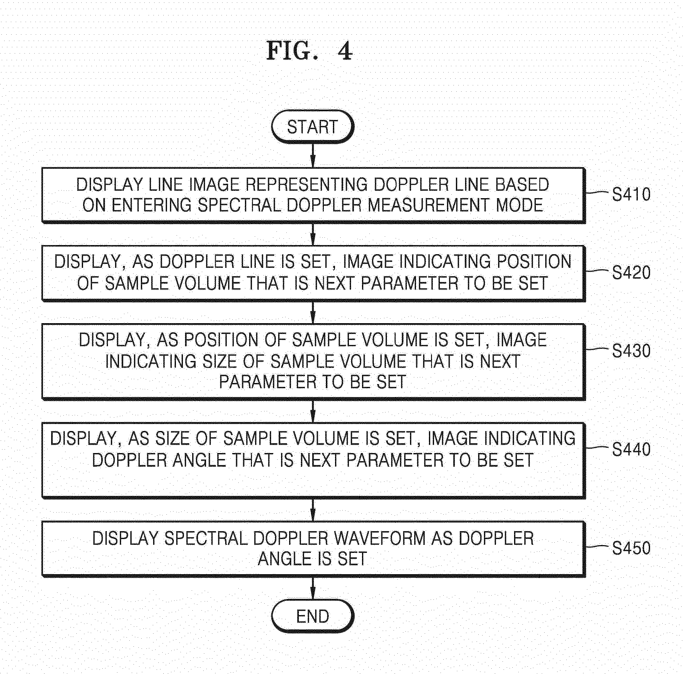

FIG. 4 is a flowchart of a method, performed by the ultrasound imaging apparatus 40, of setting Doppler parameters in a spectral Doppler measurement mode, according to an embodiment.

In operation S410, based on entering a spectral Doppler measurement mode, the ultrasound imaging apparatus 40 may display a line image representing a Doppler line.

Based on entering the spectral Doppler measurement mode, the ultrasound imaging apparatus 40 may enter a mode for setting a Doppler line without a separate user input for selecting a Doppler line among Doppler parameters. In the mode for setting a Doppler line, the ultrasound imaging apparatus 40 may display the Doppler line in the center of the screen.

In the spectral Doppler measurement mode, the ultrasound imaging apparatus 40 may set Doppler parameters according to a predetermined setting order. For example, the setting order may be preset in the ultrasound imaging apparatus 40 so that parameters are set in the order of a Doppler line, a position of a sample volume, a size of the sample volume, and a Doppler angle.

The ultrasound imaging apparatus 40 may receive a user input for moving a position of the Doppler line within an ultrasound image.

According to an embodiment, the ultrasound imaging apparatus 40 may move the ultrasound image in a lateral direction in response to receiving a user input for changing at least one of a position or an angle of the ultrasound imaging apparatus 40, without a separate user input.

According to an embodiment, the ultrasound imaging apparatus 40 may receive a user input for pressing a hardware button on a side of the ultrasound imaging apparatus 40, and in response to receiving a user input for changing at least one of the position or the angle of the ultrasound imaging apparatus 40 while the hardware button is pressed, the ultrasound imaging apparatus 40 may move the ultrasound image in the lateral direction.

Based on receiving a user input for setting the Doppler line, the ultrasound imaging apparatus 40 may determine a position of the line image (2 of FIG. 1) on the ultrasound image (10 of FIG. 1) as the Doppler line.

According to an embodiment, based on receiving a user input for selecting the software button (70 of FIG. 1), the ultrasound imaging apparatus 40 may determine the position of the line image (2 of FIG. 1) on the ultrasound image (10 of FIG. 1) as the Doppler line.

Furthermore, according to an embodiment, based on receiving a user input for releasing the pressed hardware button (50 of FIG. 1), the ultrasound imaging apparatus 40 may determine a position of the line image (2 of FIG. 1) on the ultrasound image (10 of FIG. 1) as the Doppler line.

In operation S420, as the Doppler line is set, the ultrasound imaging apparatus 40 may display an image representing the position of the sample volume, which is a next parameter to be set.

As the Doppler line is set, the ultrasound imaging apparatus 40 may display the image (6 of FIG. 1) indicating the position of the sample volume, which is a next parameter to be set among the Doppler parameters, at a center of the set Doppler line without a separate user input.

In response to receiving a user input for changing at least one of the position or angle of the ultrasound imaging apparatus 40, the ultrasound imaging apparatus 40 may move the ultrasound image (10 of FIG. 1) in the axial direction along the Doppler line.

Based on receiving a user input for setting the position of the sample volume, the ultrasound imaging apparatus 40 may determine a position of the image (6 of FIG. 1) representing the sample volume on the ultrasound image (10 of FIG. 1) as the position of the sample volume.

In operation S440, as the position of the sample volume is set, the ultrasound imaging apparatus 40 may receive a user input for adjusting a size of the sample volume, which is a next parameter to be set.

As the position of the sample volume is set, the ultrasound imaging apparatus 40 may receive a user input for adjusting the size of the sample volume, which is the next parameter to be set among the Doppler parameters, without a separate user input.

In response to receiving a user input for changing at least one of the position or angle of the ultrasound imaging apparatus 40, the ultrasound imaging apparatus 40 may change a size of the image (6 of FIG. 1) representing the sample volume in the axial direction along the Doppler line.

In response to receiving a user input for setting the size of the sample volume, the ultrasound imaging apparatus 40 may determine the size of the sample volume based on the size of the image (6 of FIG. 1) representing the sample volume.

In operation S450, as the size of the sample volume is set, the ultrasound imaging apparatus 40 may display an image representing the Doppler angle, which is a next parameter to be set.

When the size of the sample volume is set, the ultrasound imaging apparatus 40 may display the image (8 of FIG. 1) representing the Doppler angle, which is a next parameter to be set among the Doppler parameters, without a separate user input.

In response to receiving a user input for changing at least one of the position or angle of the ultrasound imaging apparatus 40, the ultrasound imaging apparatus 40 may change an angle of the image (8 of FIG. 1) representing the Doppler angle.

In response to receiving a user input for setting the Doppler angle, the ultrasound imaging apparatus 40 may determine the Doppler angle based on the angle of the image (8 of FIG. 1) representing the Doppler angle.

In operation S460, as the Doppler angle is set, the ultrasound imaging apparatus 40 may display a spectral Doppler waveform.

As the Doppler angle is set, the ultrasound imaging apparatus 40 may display the spectral Doppler waveform (80 of FIG. 1) without a separate user input.

According to an embodiment, the ultrasound imaging apparatus 40 may transmit an ultrasound signal to the object, based on the set Doppler line, the set position of the sample volume, the set size of the sample volume, and the set Doppler angle, generate the spectral Doppler waveform (80 in FIG. 1) based on an ultrasound signal reflected from the object, and display the generated spectral Doppler waveform (80 of FIG. 1).

According to an embodiment, the ultrasound imaging apparatus 40 may transmit, to another device equipped with a probe, Doppler parameter information including the set Doppler line, the set position of the sample volume, the set size of the sample volume, and the set Doppler angle, receive, from the other device, the spectral Doppler waveform (80 of FIG. 1) for the object generated based on the Doppler parameter information, and display the received spectral Doppler waveform (80 of FIG. 1).

According to an embodiment, the ultrasound imaging apparatus 40 may receive a user input for moving to the operation of setting a previous parameter or a next parameter. For example, in the operation of setting the Doppler angle, based on receiving a user input for pressing a prestored hardware button (for example, a '+' button among volume buttons), the ultrasound imaging apparatus 40 may move to the operation of setting the size of the sample volume, which is a previous parameter. In addition, based on receiving a user input for pressing a '-' button among the volume buttons in the operation of setting the size of the sample volume, the ultrasound imaging apparatus 40 may move to the operation of setting the Doppler angle, which is the next parameter following the size of the sample volume.

According to an embodiment, when entering a measurement mode, the ultrasound imaging apparatus 40 may display a UI image corresponding to a measurement parameter in a region of interest output by an artificial intelligence (Al) model. For example, the AI model may be trained to, when an ultrasound image and a Doppler parameter (e.g., the location of the sample volume) are taken as an input to the AI model, output a location of a region of interest corresponding to the Doppler parameter. Accordingly, the ultrasound imaging apparatus 40 may automatically display a UI image (e.g., an image corresponding to the position of the sample volume) corresponding to the Doppler parameter in the output region of interest among regions of the ultrasound image.

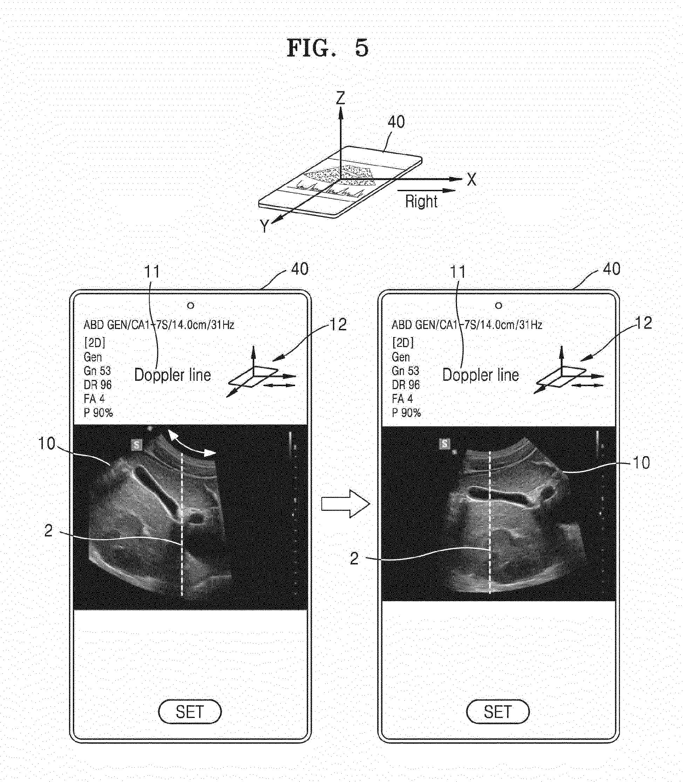

FIG. 5 illustrates a method, performed by the ultrasound imaging apparatus 40, of setting a parameter, according to an embodiment.

Referring to FIG. 5, the ultrasound imaging apparatus 40 may change a relative position of a line image 2 corresponding to a Doppler line according to movement of the ultrasound imaging apparatus 40.

In an operation of setting the Doppler line, the ultrasound imaging apparatus 40 may display, on a B-mode image 10 of an object, the line image 2 representing the Doppler line in the center of the screen.

Furthermore, the ultrasound imaging apparatus 40 may display an image or text 11 indicating that it is currently performing the operation of setting the Doppler line . In addition, the ultrasound imaging apparatus 40 may display a guide image 12 indicating a direction of movement of the ultrasound imaging apparatus 40 for moving the B-mode image 10.

According to an embodiment, in response to receiving a user input for moving the ultrasound imaging apparatus 40 to the right, the ultrasound imaging apparatus 40 may rotate the B-mode image 10 counterclockwise along the lateral direction, as shown in a diagram on the right of FIG. 5. As the B mode image 10 is rotated counterclockwise, the line image 2 may be moved to the left relative to the B mode image 10.

According to an embodiment, in response to receiving a user input for moving the ultrasound imaging apparatus 40 to the right, the ultrasound imaging apparatus 40 may move the line image 2 to the right along the lateral direction.

Based on receiving a user input for setting the Doppler line, the ultrasound imaging apparatus 40 may determine a position of the line image 2 in the B-mode image 10 as the Doppler line.

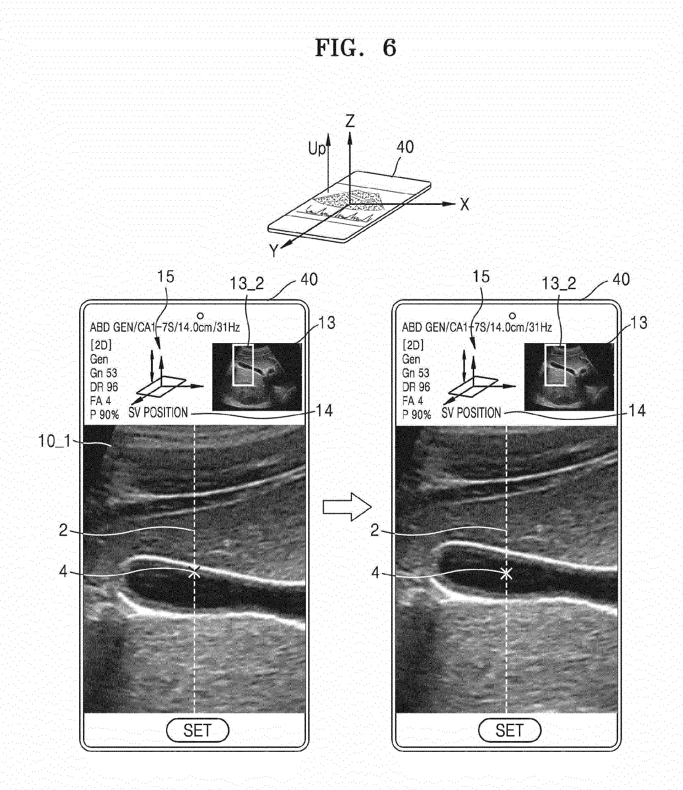

FIG. 6 illustrates a method, performed by the ultrasound imaging apparatus 40, of setting parameters, according to an embodiment.

Referring to FIG. 6, the ultrasound imaging apparatus 40 may display a position image 4 corresponding to a position of a sample volume on the line image 2 corresponding to the set Doppler line.

For example, the ultrasound imaging apparatus 40 may display the position image 4 corresponding to the position of the sample volume at the center of the line image 2.

According to an embodiment, the ultrasound imaging apparatus 40 may enlarge the B-mode image 10 around the set Doppler line and display, on an enlarged B-mode image 10_1, the line image 2 corresponding to the Doppler line and the position image 4 corresponding to the position of the sample volume. In addition, the ultrasound imaging apparatus 40 may display a reference image 13 indicating a location 13_2 of a region of the B-mode image 10 currently being displayed on the screen.

When the Doppler line is set, the B-mode image 10 is automatically enlarged around the Doppler line, so that the user may more accurately set the position of the sample volume.

The ultrasound imaging apparatus 40 may display an image or text 14 indicating that it is currently performing the operation of setting the position of the sample volume. In addition, the ultrasound imaging apparatus 40 may display a guide image 15 indicating a direction of movement of the ultrasound imaging apparatus 40 for moving the enlarged B-mode image 10_1.

According to an embodiment, in response to receiving a user input for moving the ultrasound imaging apparatus 40 upward, the ultrasound imaging apparatus 40 may move the enlarged B-mode image 10_1 upward along the Doppler line, as shown in a diagram on the right of FIG. 6. By moving the enlarged B-mode image 10_1 upward along the Doppler line, the ultrasound imaging apparatus 40 may move downward the location 13_2 of the region currently being displayed on the screen within the reference image 13.

When a position of the position image 4 does not change and the enlarged B-mode image 10_1 is moved upward, the position image 4 may appear to have been moved toward the bottom of the enlarged B-mode image 10_1 along the Doppler line.

According to an embodiment, in response to receiving a user input for moving the ultrasound imaging apparatus 40 upward, the ultrasound imaging apparatus 40 may move the position image 4 upward along the Doppler line without moving the enlarged B-mode image 10_1.

Based on receiving a user input for setting the position of the sample volume, the ultrasound imaging apparatus 40 may determine the position of the position image 4 in the enlarged B-mode image 10_1 as the position of the sample volume.

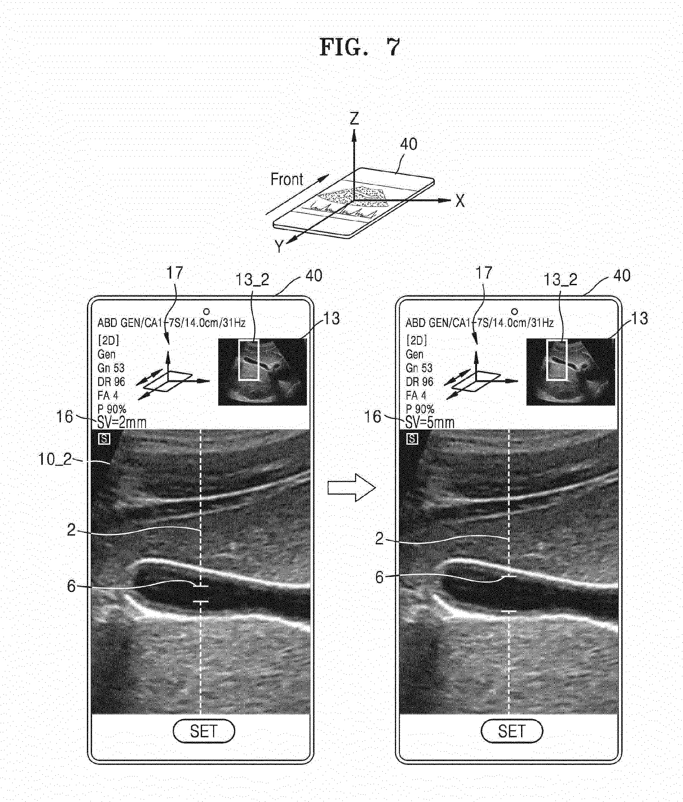

FIG. 7 illustrates a method, performed by the ultrasound imaging apparatus 40, of setting parameters, according to an embodiment.

Referring to FIG. 7, the ultrasound imaging apparatus 40 may display a size image 6 corresponding to a size of the sample volume at the set position of the sample volume.

According to an embodiment, the ultrasound imaging apparatus 40 may enlarge the B-mode image (hereinafter referred to as the ultrasound image) 10 around the position of the sample volume, and display, on an enlarged ultrasound image 10_2, the line image 2 corresponding to the Doppler line and the size image 6 corresponding to the size of the sample volume. In addition, the ultrasound imaging apparatus 40 may display the reference image 13 indicating the location 13_2 of the region of the ultrasound image 10 currently being displayed on the screen.

When the position of the sample volume is set, the ultrasound image 10 is automatically enlarged around the position of the sample volume, so the user may more accurately set the size of the sample volume.

The ultrasound imaging apparatus 40 may display a sample volume size value 16 corresponding to the currently displayed size image 6.

In addition, the ultrasound imaging apparatus 40 may display a guide image 17 indicating a direction of movement of the ultrasound imaging apparatus 40 for changing a size of the size image 6.

In response to receiving a user input for moving the ultrasound imaging apparatus 40 forward, the ultrasound imaging apparatus 40 may increase the size of the size image 6 in a direction of the Doppler line, as shown in a diagram on the right of FIG. 7.

Furthermore, in response to receiving a user input for moving the ultrasound imaging apparatus 40 backward, the ultrasound imaging apparatus 40 may reduce the size of the size image 6 in the direction of the Doppler line.

Based on receiving a user input for setting the size of the sample volume, the ultrasound imaging apparatus 40 may determine the size of the sample volume based on the size of the size image 6.

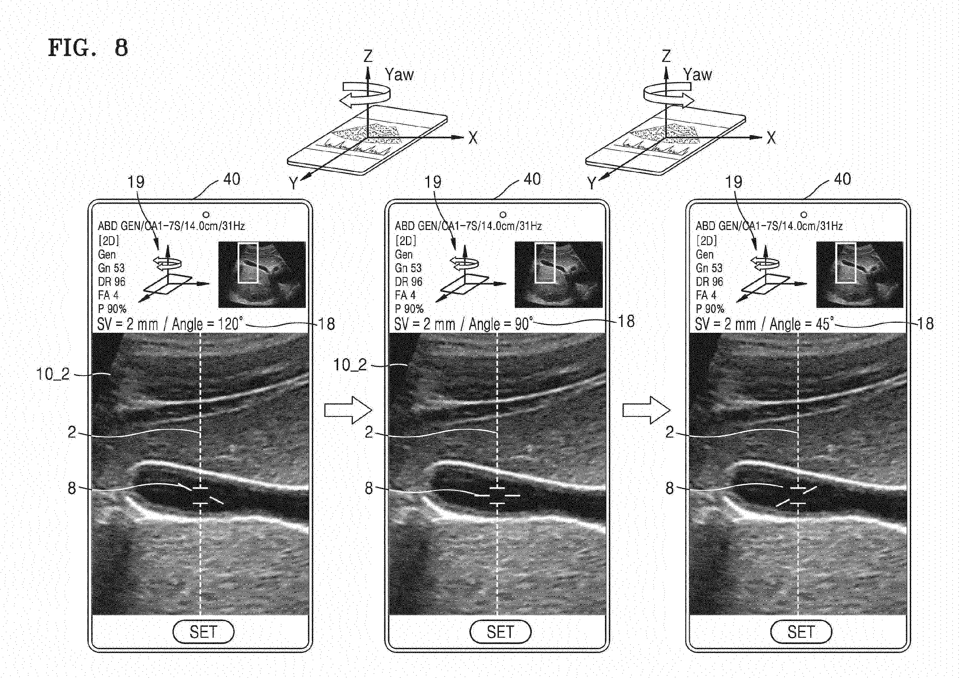

FIG. 8 illustrates a method, performed by the ultrasound imaging apparatus 40, of setting parameters, according to an embodiment.

Referring to FIG. 8, the ultrasound imaging apparatus 40 may display a Doppler angle image 8 at the set position of the sample volume.

The ultrasound imaging apparatus 40 may display a Doppler angle value 18 corresponding to an angle of the currently displayed angle image 8.

In addition, the ultrasound imaging apparatus 40 may display a guide image 19 indicating a direction of movement of the ultrasound imaging apparatus 40 for changing the angle of the angle image 8.

In response to receiving a user input for rotating the ultrasound imaging apparatus 40 clockwise about the Z axis, the ultrasound imaging apparatus 40 may rotate the angle image 8 clockwise, as shown in a first diagram of FIG. 8.

In response to receiving a user input for rotating the ultrasound imaging apparatus 40 counterclockwise about the Z axis, the ultrasound imaging apparatus 40 may rotate the angle image 8 counterclockwise, as shown in a third diagram of FIG. 8.

In response to receiving a user input for setting a Doppler angle, the ultrasound imaging apparatus 40 may determine a Doppler angle value based on a slope of the angle image 8. For example, the ultrasound imaging apparatus 40 may determine an angle formed by the angle image 8 and the Doppler line as a Doppler angle value.

According to an embodiment, when the Doppler angle is determined, the ultrasound imaging apparatus 40 may transmit an ultrasound signal to the object, based on the set Doppler line, the set position of the sample volume, the set size of the sample volume, and the set Doppler angle without separate user input, without a separate user input, and generate a spectral Doppler image (e.g., the spectral Doppler waveform 80 of FIG. 1) based on an ultrasound signal reflected from the object. In addition, the ultrasound imaging apparatus 40 may display the generated spectral Doppler image (the spectral Doppler waveform 80 of FIG. 1).

According to an embodiment, when the Doppler angle is determined, the ultrasound imaging apparatus 40 may transmit, to another device equipped with a probe, Doppler parameter information including the set Doppler line, the set position of the sample volume, the set size of the sample volume, and the set Doppler angle without a separate user input, receive, from the other device, the spectral Doppler image (the spectral Doppler waveform 80 of FIG. 1) of the object generated based on the Doppler parameter information, and display the received spectral Doppler image.

According to an embodiment, as all the Doppler parameters are set, the ultrasound imaging apparatus 40 may change the enlarged ultrasound image 10_2 to the original ultrasound image 10. For example, the ultrasound imaging apparatus 40 may delete the enlarged ultrasound image 10_2 when the Doppler angle is determined, and display the UI images, i.e., the line image2, the size image 6, and the angle image 8, corresponding to the set parameters on the unenlarged ultrasound image 10 before the Doppler parameters are set.

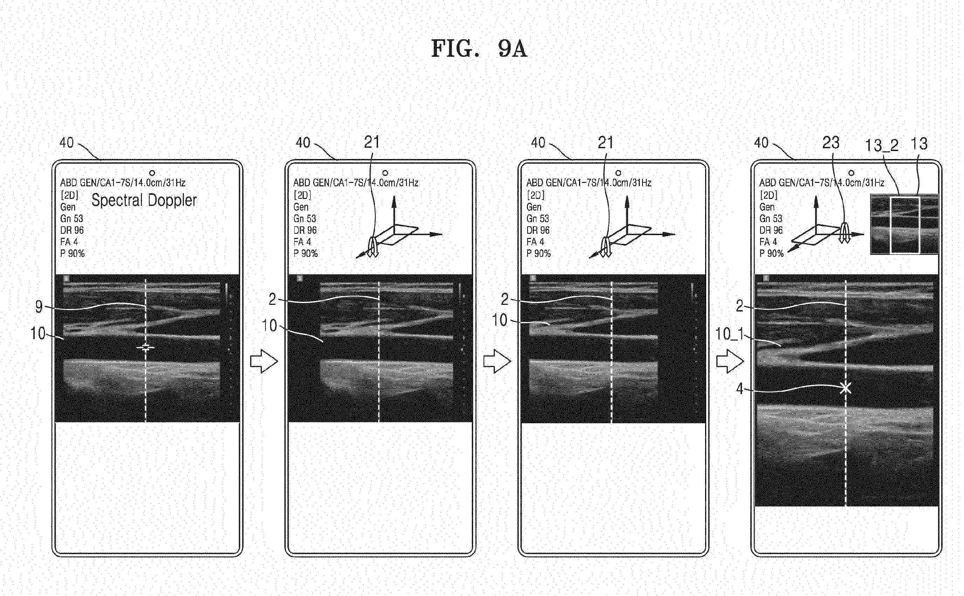

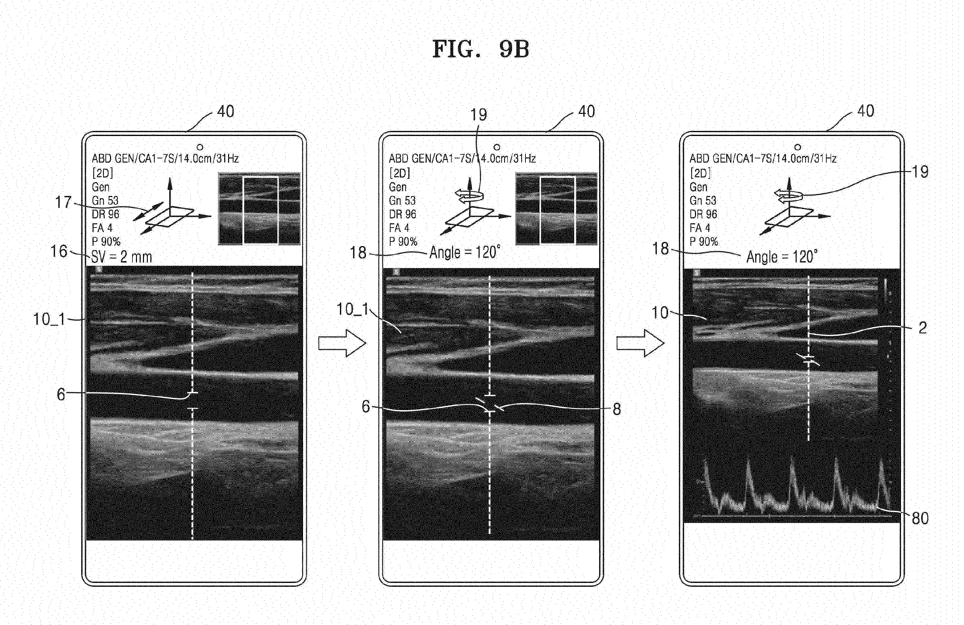

FIGS. 9A and 9B illustrate a method, performed by the ultrasound imaging apparatus 40, of setting parameters on an ultrasound image by using a linear probe, according to an embodiment.

Referring to a first diagram of FIG. 9A, the ultrasound imaging apparatus 40 may display an ultrasound image 10 obtained using a linear probe. Furthermore, in response to receiving a user input for selecting a spectral Doppler mode, the ultrasound imaging apparatus 40 may display UI images 9 representing spectral Doppler parameters on the ultrasound image 10. In addition, the ultrasound imaging apparatus 40 may display text indicating that it is in a spectral Doppler mode.

Referring to a second diagram of FIG. 9A, the ultrasound imaging apparatus 40 may proceed to an operation of setting a Doppler line.

According to an embodiment, after displaying the UI images 9 representing the spectral Doppler parameters for a predetermined time (e.g., 2 seconds), the ultrasound imaging apparatus 40 may proceed to the operation of setting the Doppler line without a separate user input.

In the operation of setting the Doppler line, the ultrasound imaging apparatus 40 may display a line image 2 corresponding to the Doppler line on the ultrasound image 10. In addition, the ultrasound imaging apparatus 40 may display a guide image 21 that guides movement of the ultrasound imaging apparatus 40 for moving the ultrasound image 10.

Based on receiving a user input for rotating the ultrasound imaging apparatus 40 counterclockwise about the Y axis, the ultrasound imaging apparatus 40 may move the ultrasound image 10 to the left.

Referring to a third diagram of FIG. 9A, as the ultrasound image 10 is moved to the left while a position of the line image 2 is fixed, the line image 2 may be moved to the right relative to the ultrasound image 10.

Based on receiving a user input for setting the Doppler line, the ultrasound imaging apparatus 40 may determine the position of the line image 2 on the ultrasound image 10 as the Doppler line.

Referring to a fourth diagram of FIG. 9A, when the Doppler line is determined, the ultrasound imaging apparatus 40 may proceed to an operation of setting a position of a sample volume without a separate user input.

When proceeding to the operation of setting the position of the sample volume, the ultrasound imaging apparatus 40 may enlarge the ultrasound image 10 around the set Doppler line. Furthermore, as the ultrasound imaging apparatus 40 enlarges the ultrasound image 10, the ultrasound imaging apparatus 40 may display only a region of the ultrasound image 10. Furthermore, the ultrasound imaging apparatus 40 may display a reference image 13 indicating a location 13_2 of the region of the ultrasound image 10 that is currently being displayed on the screen.

Furthermore, when proceeding to the operation of setting the position of the sample volume, the ultrasound imaging apparatus 40 may display a position image 4 corresponding to the position of the sample volume on the set Doppler line. In addition, the ultrasound imaging apparatus 40 may display a guide image 23 that guides movement of the ultrasound imaging apparatus 40 for moving the enlarged ultrasound image 10.

Based on receiving a user input for rotating the ultrasound imaging apparatus 40 clockwise about the X axis, the ultrasound imaging apparatus 40 may move the ultrasound image 10 upward.

Based on receiving a user input for setting the position of the sample volume, the ultrasound imaging apparatus 40 may determine a position of the position image 4 within the enlarged ultrasound image 10_1 as the position of the sample volume.

Referring to a first diagram of FIG. 9B, when the position of the sample volume is determined, the ultrasound imaging apparatus 40 may proceed to an operation of setting a size of the sample volume without a separate user input.

When proceeding to the operation of setting the size of the sample volume, the ultrasound imaging apparatus 40 may display a size image 6 corresponding to the size of the sample volume at the set position of the sample volume. Furthermore, the ultrasound imaging apparatus 40 may display a guide image 17 guiding movement of the ultrasound imaging apparatus 40 for adjusting a size of the size image 6.

In response to receiving a user input for moving the ultrasound imaging apparatus 40 forward, the ultrasound imaging apparatus 40 may increase the size of the size image 6 along the Doppler line. In addition, the ultrasound imaging apparatus 40 may display text 16 indicating the size of the sample volume corresponding to the size of the size image 6.

In response to receiving a user input for setting the size of the sample volume, the ultrasound imaging apparatus 40 may determine the size of the sample volume based on the size of the size image 6.

Referring to a second diagram of FIG. 9B, when the size of the sample volume is determined, the ultrasound imaging apparatus 40 may proceed to an operation of setting a Doppler angle without a separate user input.

When proceeding to the operation of setting the Doppler angle, the ultrasound imaging apparatus 40 may display a Doppler angle image 8 at the set position of the sample volume.

The ultrasound imaging apparatus 40 may display an image or text 18 indicating a size of the set Doppler angle.

In addition, the ultrasound imaging apparatus 40 may display a guide image 19 indicating a direction of movement of the ultrasound imaging apparatus 40 for rotating the angle image 8.

In response to receiving a user input for rotating the ultrasound imaging apparatus 40 clockwise about the Z axis, the ultrasound imaging apparatus 40 may rotate the angle image 8 clockwise.

In response to receiving a user input for setting a Doppler angle, the ultrasound imaging apparatus 40 may determine the Doppler angle based on a slope of the angle image 8. For example, the ultrasound imaging apparatus 40 may determine an angle formed between the angle image 8 and the Doppler line as the Doppler angle.

According to an embodiment, when the Doppler angle is determined, the ultrasound imaging apparatus 40 may transmit an ultrasound signal to the object based on the set Doppler line, the set position of the sample volume, the set size of the sample volume, and the set Doppler angle, generate a spectral Doppler image 80 based on an ultrasound signal reflected from the object, and display the generated spectral Doppler image 80.

According to an embodiment, when the Doppler angle is determined, the ultrasound imaging apparatus 40 may transmit, to another device equipped with a probe, Doppler parameter information including the set Doppler line, the set position of the sample volume, the set size of the sample volume, and the set Doppler angle. Furthermore, the ultrasound imaging apparatus 40 may receive, from the other device, spectral Doppler information (e.g., the spectral Doppler image 80) about the object generated based on the Doppler parameter information, and display the received spectral Doppler information.

According to an embodiment, when the Doppler angle is determined, the ultrasound imaging apparatus 40 may determine that the measurement parameters corresponding to the measurement item are all set, and reduce the enlarged ultrasound image 10_1 to the original ultrasound image 10.

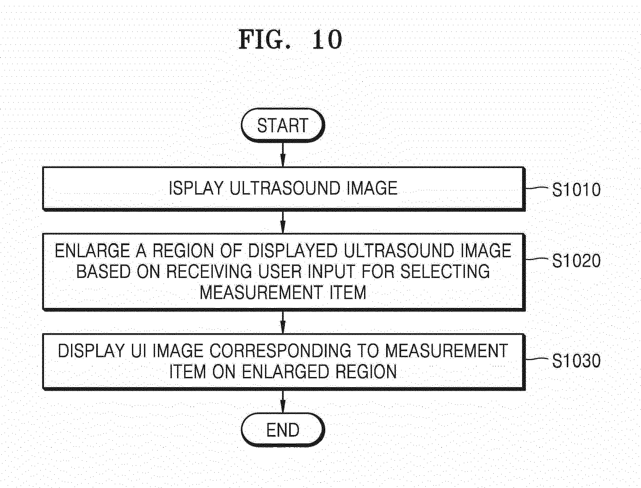

FIG. 10 is a flowchart of a method, performed by the ultrasound imaging apparatus 40, of enlarging a region of an ultrasound image as it enters a measurement mode, according to an embodiment.

In operation S1010, the ultrasound imaging apparatus 40 may display an ultrasound image.

In operation S1020, the ultrasound imaging apparatus 40 may enlarge a region of the displayed ultrasound image based on receiving a user input for selecting a measurement item. Examples of the measurement item may include, but are not limited to, distance measurement, area measurement, and spectral Doppler waveform measurement.

According to an embodiment, the ultrasound imaging apparatus 40 may enlarge the displayed ultrasound image and determine a region of the entire enlarged ultrasound image to be displayed. For example, the ultrasound imaging apparatus 40 may include an AI model that is trained to output a region in the ultrasound image when an ultrasound image and a measurement item are input to the AI model.

To this end, the AI model may be trained based on a large amount of training data. One training data may include an ultrasound image and a measurement item as input data of the training data and a region selected by the user within the ultrasound image as output data for the training data. For example, when the ultrasound image is an ultrasound image of a carotid artery and the measurement item is a spectral Doppler waveform, the AI model may be trained to output a location of a carotid artery region in the ultrasound image when the ultrasound image of the carotid artery and the spectral Doppler waveform are input thereto.

Accordingly, the ultrasound imaging apparatus 40 may receive the displayed ultrasound image and the measurement item as an input to the AI model, and enlarge the region output from the AI model to be displayed.

In addition, the ultrasound imaging apparatus 40 may display a reference image indicating a location of a region to be enlarged and displayed within the ultrasound image.

In operation S1030, the ultrasound imaging apparatus 40 may display a UI image, corresponding to the measurement item, on the enlarged region.

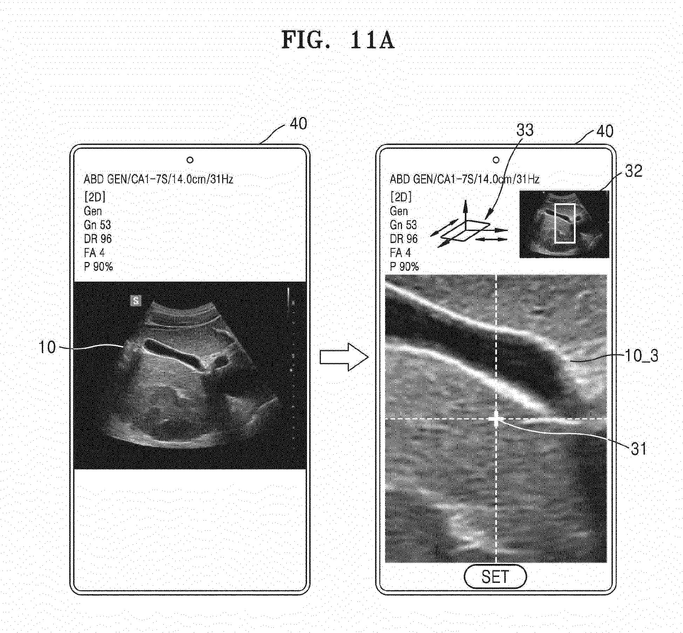

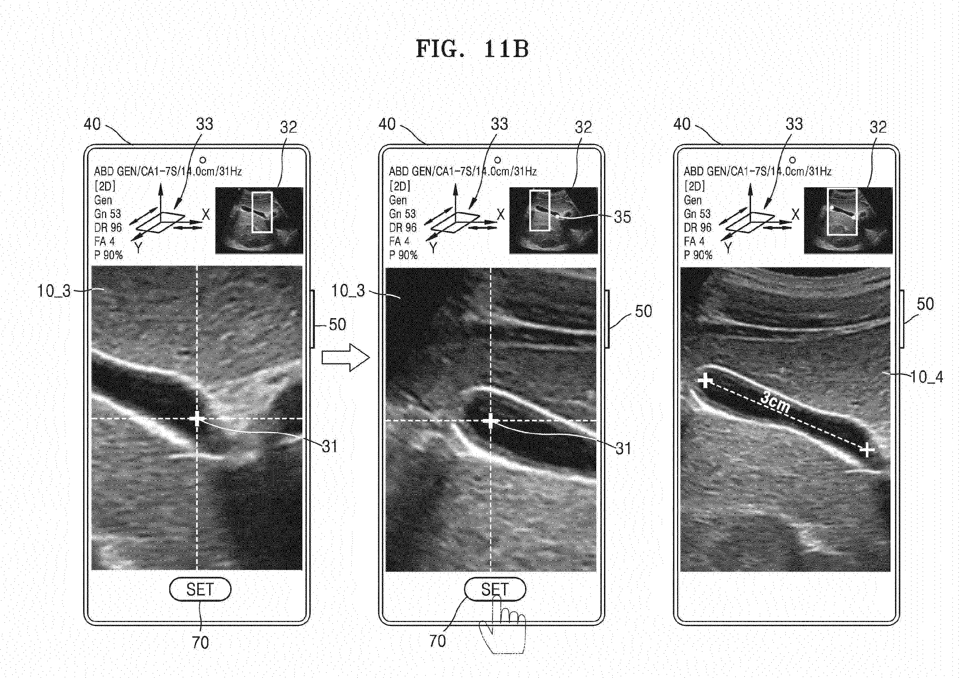

FIGS. 11A and 11B illustrate a method, performed by the ultrasound imaging apparatus 40, of providing a user interface for measuring a distance, according to an embodiment.

Referring to FIGS. 11A and 11B, the ultrasound imaging apparatus 40 may provide a user interface for distance measurement.

The ultrasound imaging apparatus 40 may receive a user input for selecting a distance measurement item.

For example, the ultrasound imaging apparatus 40 may display a software button (e.g., a distance measurement menu) corresponding to a distance measurement item, and receive a user input for selecting the software button.

Furthermore, the ultrasound imaging apparatus 40 may receive a user input for selecting a hardware button corresponding to the distance measurement item. For example, when an ultrasound image 10 is displayed, the ultrasound imaging apparatus 40 may enter a distance measurement mode based on receiving a user input for pressing a volume button.

In addition, based on receiving a user input for shaking the ultrasound imaging apparatus 40, the ultrasound imaging apparatus 40 may enter the distance measurement mode.

Referring to a diagram on the right of FIG. 11A, in response to receiving a user input for selecting the distance measurement item, the ultrasound imaging apparatus 40 may enlarge and display a region of the displayed ultrasound image 10.

Furthermore, the ultrasound imaging apparatus 40 may display a UI image 31 representing a measurement point for distance measurement on an enlarged ultrasound image 10_3. For example, the ultrasound imaging apparatus 40 may display the UI image 31 representing the measurement point in the center of the enlarged ultrasound image 10_3.

Furthermore, the ultrasound imaging apparatus 40 may display guide information 33 that guides movement of the ultrasound imaging apparatus 40 for moving the enlarged ultrasound image 10_3. In addition, the ultrasound imaging apparatus 40 may display a reference image 32 indicating a location of a region being enlarged and displayed in the entire area of the ultrasound image 10.

Referring to FIG. 11B, the ultrasound imaging apparatus 40 may display the ultrasound image 10 so that, as the user moves the ultrasound imaging apparatus 40 in the X-Y plane, a magnified version of the ultrasound image 10 enlarged via a magnifying glass is displayed on the screen of the ultrasound imaging apparatus 40.

For example, referring to a first diagram of FIG. 11B, based on receiving a user input for moving the ultrasound imaging apparatus 40 to an upper right corner in the X-Y plane, the ultrasound imaging apparatus 40 may display an upper right region of the enlarged ultrasound image 10_3 relative to the currently displayed region (the enlarged region of the ultrasound image 10 shown in the right diagram of FIG. 11A). Because the position of the UI image 31 representing the measurement point is fixed, the UI image 31 representing the measurement point may also be moved to the upper right of the enlarged ultrasound image 10_3.

Based on receiving a user input for setting a first measurement point, the ultrasound imaging apparatus 40 may determine a position of the UI image 31 on the enlarged ultrasound image 10_3 as the first measurement point. The user input for setting the first measurement point may be, for example, a user input for selecting a software button 70 or a hardware button 50 for setting a measurement point.

Referring to a second diagram of FIG. 11B, when the first measurement point is determined, the ultrasound imaging apparatus 40 may display, on the reference image 35, an image 35 indicating a position of the set first measurement point.

Again, based on receiving a user input for moving the ultrasound imaging apparatus 40 to an upper left corner in the X-Y plane, the ultrasound imaging apparatus 40 may display an upper left region of the enlarged ultrasound image 10_3 relative to the currently displayed region (the region of the enlarged ultrasound image 10_3 shown in the first diagram of FIG. 11B). The UI image 31 representing the measurement point may also be moved to the upper left of the enlarged ultrasound image 10_3.

Based on receiving a user input for setting a second measurement point, the ultrasound imaging apparatus 40 may determine a position of the UI image 31 on the enlarged ultrasound image 10_3 as the first measurement point.

Referring to a third diagram of FIG. 11B, when the second measurement point is determined, the ultrasound imaging apparatus 40 may calculate a distance between the first measurement point and the second measurement point. Furthermore, the ultrasound imaging apparatus 40 may display the calculated distance.

When the distance between the first measurement point and the second measurement point is calculated, the ultrasound imaging apparatus 40 may reduce the enlarged ultrasound image 10_3. For example, the ultrasound imaging apparatus 40 may reduce the enlarged ultrasound image 10_3 to the extent that the first measurement point and the second measurement point may be displayed together on a single screen. Furthermore, the ultrasound imaging apparatus 40 may display the first measurement point, the second measurement point, and the calculated distance on a reduced ultrasound image 10_4.

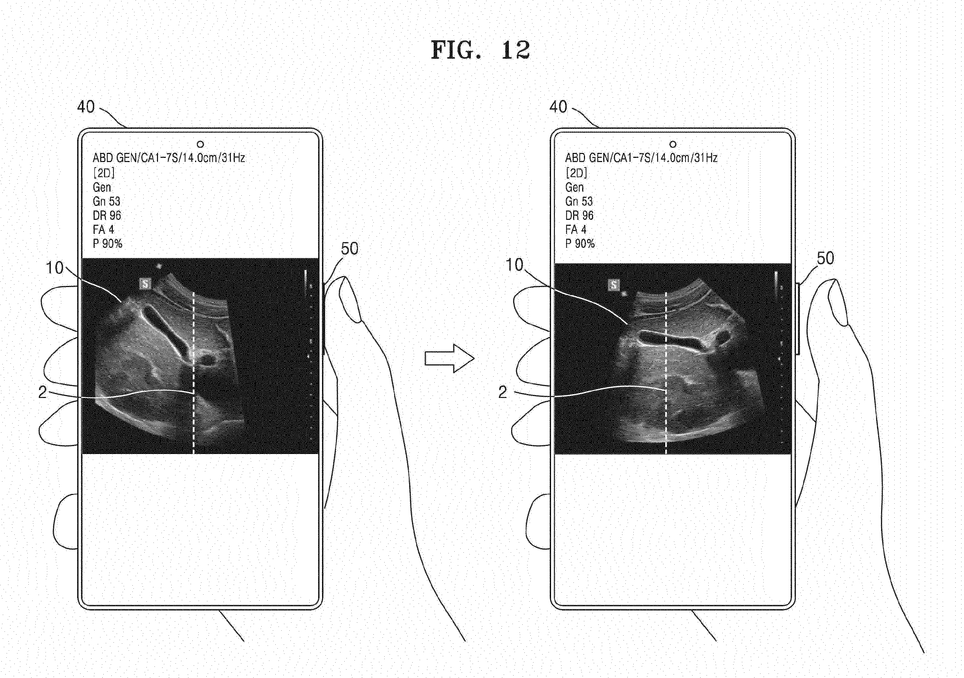

FIG. 12 illustrates a method, performed by the ultrasound imaging apparatus 40, of setting a measurement parameter, according to an embodiment.

Referring to FIG. 12, the ultrasound imaging apparatus 40 may receive a user input for changing at least one of a position and an angle of the ultrasound imaging apparatus 40 only when a user input for pressing a predetermined hardware button is received.

In response to receiving a user input for changing at least one of the position and angle of the ultrasound imaging apparatus 40 while the predetermined hardware button is pressed, the ultrasound imaging apparatus 40 may change a position of a UI image within an ultrasound image 10 or a position of the ultrasound image 10.

For example, referring to a diagram on the left of FIG. 12, in an operation of setting a Doppler line, the ultrasound imaging apparatus 40 may detect whether the volume button 50 is pressed when a user input for moving the ultrasound imaging apparatus 40 is received.

Based on detecting that the volume button 50 is pressed, the ultrasound imaging apparatus 40 may change a position of the line image 2 corresponding to the Doppler line or change the position of the ultrasound image 10 according to the movement of the ultrasound imaging apparatus 40.

In response to receiving a user input for releasing the pressed hardware button, the ultrasound imaging apparatus 40 may determine the position of the UI image within the ultrasound image 10 as a value of a measurement parameter.

For example, referring to a diagram on the right of FIG. 12, in response to receiving a user input for releasing the volume button 50, the ultrasound imaging apparatus 40 may determine the position of the line image 2 on the ultrasound image 10 as the Doppler line.

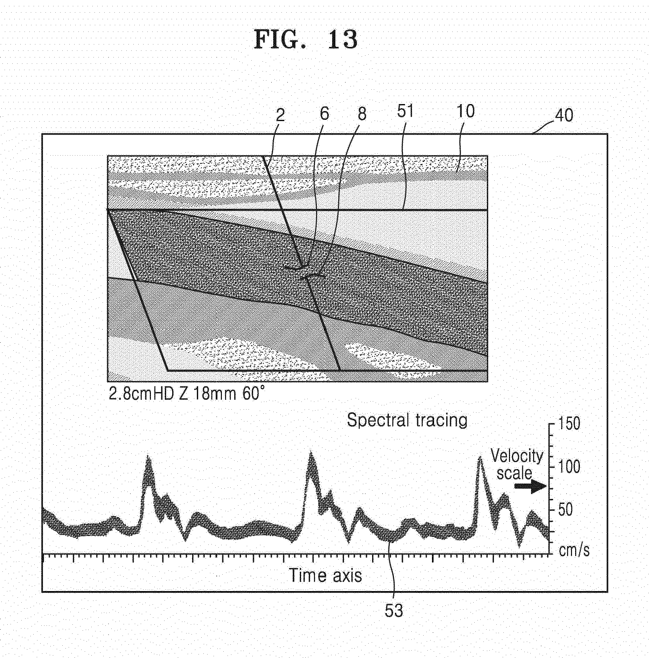

FIG. 13 illustrates a method, performed by the ultrasound imaging apparatus 40, of setting Doppler parameters, according to an embodiment.

Referring to FIG. 13, the ultrasound imaging apparatus 40 may display a color Doppler image 10. Furthermore, the ultrasound imaging apparatus 40 may determine a Doppler line based on a Doppler line image 2. Furthermore, the ultrasound imaging apparatus 40 may determine a position and a size of a sample volume, based on a position and a size of a sample volume image 6. In addition, the ultrasound imaging apparatus 40 may determine an angle formed between a Doppler angle image 8 and the Doppler line image 2 as a Doppler angle.

The ultrasound imaging apparatus 40 may obtain, based on the determined Doppler line, sample volume, and Doppler angle, a spectral Doppler waveform 53 indicating velocity of blood flow in the sample volume of the object, and display the obtained spectral Doppler waveform 53.

A spectral Doppler waveform is a representation of Doppler information of blood flow on the X- and Y-axes by using fast Fourier transform (FFT). The X-axis of the spectral Doppler waveform 53 represents time, and the Y-axis represents the velocity at the sample volume which is at a point of measurement.

The Doppler line may refer to a scan line over which Doppler information is to be measured. The position of the sample volume may also refer to a depth at which the Doppler information is to be measured within the Doppler line. The size of the sample volume may also refer to an interval at which a Doppler signal is to be measured at the position of the sample volume. The Doppler angle may also refer to an angle formed between a direction of blood flow and an ultrasound beam.

The ultrasound imaging apparatus 40 may transmit an ultrasound signal along the Doppler line and receive an ultrasound signal reflected from the depth of the sample volume. The ultrasound imaging apparatus 40 may obtain a Doppler shift f_{D} which is a difference between a frequency fᵣ of the received ultrasound signal and a frequency f₀ of a transmitted ultrasound signal. By applying the obtained Doppler shift f_{D}, the frequency f₀ of the transmitted ultrasound signal, and the Doppler angle to a Doppler equation, the ultrasound imaging apparatus 40 may calculate a velocity of red blood cells at the position of the sample volume of the object.

The ultrasound imaging apparatus 40 may calculate the velocity of red blood cells at the position of the sample volume over time. Furthermore, the ultrasound imaging apparatus 40 may display the velocity of the red blood cells over time as the spectral Doppler waveform 53.

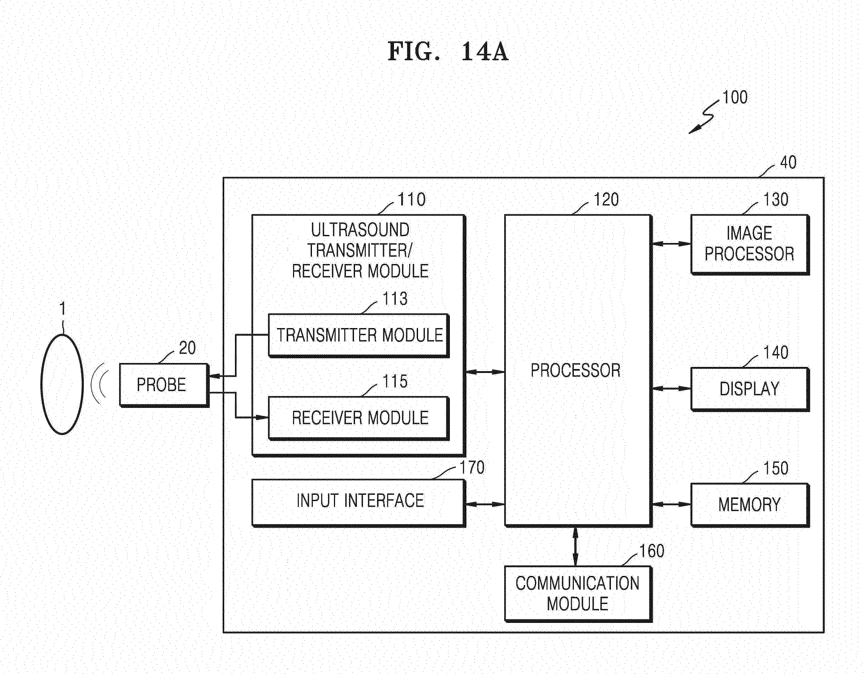

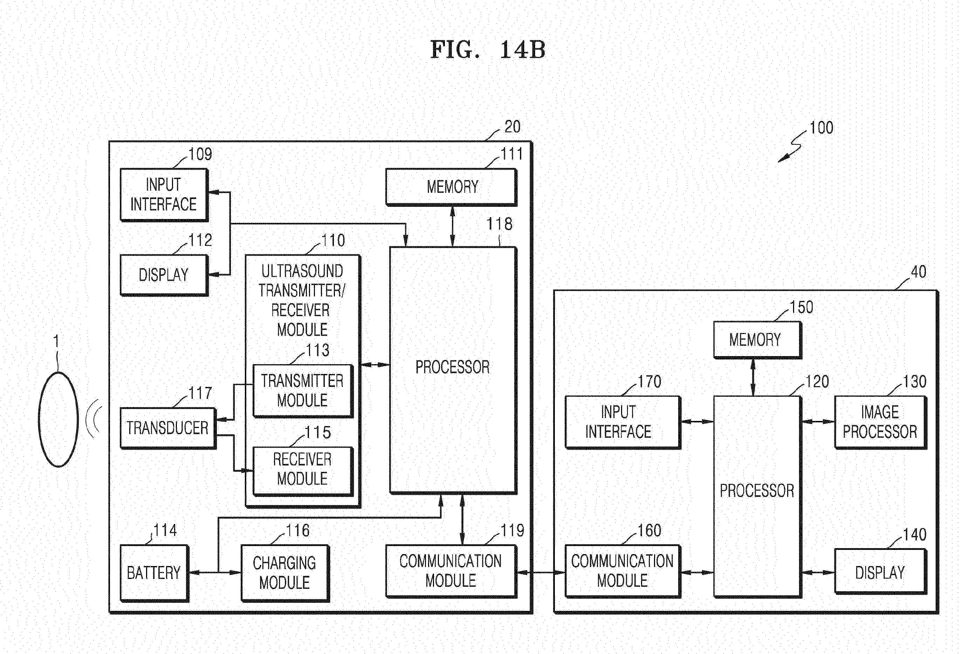

FIGS. 14A and 14B are block diagrams of a configuration of an ultrasound imaging system according to an embodiment.

Referring to FIGS. 14A and 14B, an ultrasound imaging system 100 may include a probe 20 and an ultrasound imaging apparatus 40.

The ultrasound imaging apparatus 40 may be implemented not only as a cart-type ultrasound imaging apparatus but also as a portable ultrasound imaging apparatus. Examples of the portable ultrasound imaging apparatus may include, but are not limited to, a smartphone, a laptop computer, a personal digital assistant (PDA), a tablet personal computer (PC), etc., each of which includes a probe and an application. The ultrasound imaging apparatus 40 may be formed integrally with the probe 20.

The probe 20 may include a wired probe that is connected to the ultrasound imaging apparatus 40 by wire to communicate with the ultrasound imaging apparatus 40 by wire, a wireless probe that is wirelessly connected to the ultrasound imaging apparatus 40 to communicate wirelessly with the ultrasound imaging apparatus 40, and/or a hybrid probe that is connected to the ultrasound imaging apparatus 40 by wire or wirelessly to communicate with the ultrasound imaging apparatus 40 by wire or wirelessly.

According to various embodiments, the ultrasound imaging apparatus 40 may include an ultrasound transmitter/receiver module 110 as shown in FIG. 14A, or the probe 20 may include the ultrasound transmitter/receiver module 110 as shown in FIG. 14B. According to various embodiments, the ultrasound imaging apparatus 40 and the probe 20 may both include the ultrasound transmitter/receiver module 110.

According to various embodiments, the probe 20 may further include at least one of an image processor 130, a display 140, or an input interface 170, or a combination thereof. In the disclosure, descriptions of the ultrasound transmitter/receiver module 110, the image processor 130, the display 140, or the input interface 170 included in the ultrasound imaging apparatus 40 may also apply to the ultrasound transmitter/receiver module 110, the image processor 130, the display 140, or the input interface 170 included in the probe 20.

FIG. 14A is a block diagram of a configuration of the ultrasound imaging system 100 when the probe 20 is a wired probe or a hybrid probe.

The probe 20 may include a plurality of transducer elements. The plurality of transducer elements are arranged in a predetermined array, forming a transducer array. The transducer array may correspond to a one-dimensional (1D) array or a two-dimensional (2D) array. The plurality of transducer elements may transmit ultrasound signals to an object 1 in response to transmission signals applied from a transmitter module 113. The plurality of transducer elements may receive ultrasound (echo) signals reflected from the object 1 to form reception signals. Furthermore, the probe 20 may be formed integrally with the ultrasound imaging apparatus 40, or may be implemented as a separate part connected to the ultrasound imaging apparatus 40 in a wired manner. In addition, the ultrasound imaging apparatus 40 may be connected to one or a plurality of probes 20 according to its implemented configuration.

When the probe 20 is a wired probe or hybrid probe, the probe 20 may include a cable and a connector that are connectable to a connector of the ultrasound imaging apparatus 40.

According to an embodiment, the probe 20 may be implemented as a 2D probe. When the probe 20 is implemented as a 2D probe, the plurality of transducer elements included in the probe 20 may be arranged in two dimensions to form a 2D transducer array.

For example, the 2D transducer array may include a plurality of sub-arrays, each of the plurality of sub-arrays including a plurality of transducer elements arranged in a first direction, wherein the plurality of sub-arrays are arranged in a second direction that is different from the first direction.

Furthermore, according to an embodiment, when the probe 20 is implemented as a 2D probe, the ultrasound transmitter/receiver module 110 may include at least one of an analog beamformer or a digital beamformer. Further, according to an embodiment, the 2D probe may include at least one of an analog beamformer or a digital beamformer, or a combination thereof, according to its implemented configuration.

The processor 120 may control the transmitter module 113 to form transmission signals to be respectively applied to the plurality of transducer elements based on positions and a focal point of the plurality of transducer elements.

The processor 120 may control the receiver module 115 to perform analog-to-digital conversion (ADC) on the reception signals received from the probe 20 and generate ultrasound data by summing the digital reception signals based on positions and a focal point of the plurality of transducer elements.

When the probe 20 is implemented as a 2D probe, the processor 120 may calculate a time delay value for digital beamforming with respect to each of the plurality of sub-arrays included in the 2D transducer array. Also, the processor 120 may calculate a time delay value for analog beamforming for each of the plurality of transducer elements included in any one of the plurality of sub-arrays. The processor 120 may control the analog beamformer and the digital beamformer to form a transmission signal to be applied to each of the plurality of transducer elements based on time delay values for analog beamforming and digital beamforming. The processor 120 may also control the analog beamformer to sum signals received from the plurality of transducer elements for each sub-array according to the time delay values for analog beamforming. Furthermore, the processor 120 may control the ultrasound transmitter/receiver module 110 to perform ADC on the resulting sum signal for each sub-array. In addition, the processor 120 may control the digital beamformer to generate ultrasound data by summing the digital output signals according to the time delay values for digital beamforming.

The image processor 130 generates or processes an ultrasound image by using the generated ultrasound data.

The display 140 may display the generated ultrasound image and various pieces of information processed by the ultrasound imaging apparatus 40 or the probe 20. The probe 20 or the ultrasound imaging apparatus 40 may include one or a plurality of displays 140 depending on its implemented configuration. Furthermore, the display 140 may include a touch panel or a touch screen. The display 140 may also include a flexible display.

The processor 120 may control all operations of the ultrasound imaging apparatus 40 and operations of components of the ultrasound imaging apparatus 40. The processor 120 may execute programs or instructions stored in the memory 150 to perform or control various operations or functions of the ultrasound imaging apparatus 40. The processor 120 may also receive a control signal from the input interface 170 or an external device to control an operation of the ultrasound imaging apparatus 40.

The ultrasound imaging apparatus 40 includes the communication module 160 via which it may be connected to and communicate with external devices (e.g., servers, medical devices, and portable devices such as smartphones, tablet PCs, wearable devices, etc.).

The communication module 160 may include at least one component that enables communication with an external device. The communication module 160 may include, for example, at least one of a short-range communication module, a wired communication module, or a wireless communication module.

The communication module 160 may receive a control signal and data from an external device. The processor 120 may control an operation of the ultrasound imaging apparatus 40 in response to the control signal received via the communication module 160. Furthermore, the processor 120 may transmit a control signal to an external device via the communication module 160 to control the external device in response to the transmitted control signal. The external device may operate in response to a control signal received from the ultrasound imaging apparatus 40, or process data received from the ultrasound imaging apparatus 40.

A program or application related to the ultrasound imaging apparatus 40 may be installed on the external device. The program or application installed on the external device may control the ultrasound imaging apparatus 40, or run in response to a control signal or data received from the ultrasound imaging apparatus 40.

The external device may receive or download the program or application related to the ultrasound imaging apparatus 40 from the ultrasound imaging apparatus 40, the probe 20, or a server, and install and execute the program or application thereon. The ultrasound imaging apparatus 40, the probe 20, or the server providing a program or application may include a recording medium storing instructions, commands, installation files, executable files, or related data of the program or application. The external device may also be sold with programs or applications installed.

The memory 150 may store various types of data or programs for driving and controlling the ultrasound imaging apparatus 40, input and/or output ultrasound data, ultrasound images, etc.

The input interface 170 may receive a user input for controlling the ultrasound imaging apparatus 40. For example, the user input may include, but is not limited to, inputs for manipulating buttons, keypads, mice, trackballs, jog switches, or knops, an input for touching a touchpad or a touch screen, a voice input, a motion input, and an input of biometric information (e.g., iris recognition, fingerprint recognition, etc.).

FIG. 14B is a control block diagram of a configuration of the ultrasound imaging system 100 when the probe 20 is a wireless probe or a hybrid probe.

According to various embodiments, the ultrasound imaging apparatus 40 shown in FIG. 14B may be replaced with the ultrasound imaging apparatus 40 described with reference to FIG. 14A.

According to various embodiments, the probe shown in FIG. 14A may be replaced with the probe 20 described with reference to FIG. 14B.

The probe 20 may include a display 112, a transmitter module 113, a battery 114, a transducer 117, a charging module 116, a receiver module 115, an input interface 109, a processor 118, and a communication module 119. Although FIG. 14B shows that the probe 20 includes both the transmitter module 113 and the receiver module 115, according to its implemented configuration, the probe 20 may include only some of the components of the transmitter module 113 and the receiver module 115, and the ultrasound imaging apparatus 40 may also include some of the components of the transmitter module 113 and the receiver module 115. In addition, the probe 20 may further include the image processor 130.

The transducer 117 may include a plurality of transducer elements. The plurality of transducer elements are arranged in a predetermined array, forming a transducer array. The transducer array may correspond to a 1D array or a 2D array. The plurality of transducer elements may transmit ultrasound signals to an object 1 in response to transmission signals applied from the transmitter module 113. Furthermore, the plurality of transducer elements may receive ultrasound signals reflected from the object 1 to form or generate electrical reception signals.

The charging module 116 may charge the battery 114. The charging module 116 may receive power from an external source. According to an embodiment, the charging module 116 may receive power wirelessly. Furthermore, according to an embodiment, the charging module 116 may receive power by wire. The charging module 116 may transmit the received power to the battery 114.

The processor 118 may control the transmitter module 113 to generate or form transmission signals to be respectively applied to the plurality of transducer elements, based on positions and a focal point of the plurality of transducer elements.

The processor 118 may control the receiver module 115 to perform ADC on the reception signals received from the transducer 117 and generate ultrasound data by summing the digital reception signals based on positions and a focal point of the plurality of transducer elements. According to an embodiment, when the probe 20 includes the image processor 130, the image processor 130 may generate an ultrasound image based on the generated ultrasound data.

When the probe 20 is implemented as a 2D probe, the processor 118 may calculate a time delay value for digital beamforming with respect to each of the plurality of sub-arrays included in the 2D transducer array. Also, the processor 118 may calculate a time delay value for analog beamforming for each of the plurality of transducer elements included in any one of the plurality of sub-arrays. The processor 118 may control an analog beamformer and a digital beamformer to form a transmission signal to be applied to each of the plurality of transducer elements based on time delay values for analog beamforming and digital beamforming. The processor 118 may also control the analog beamformer to sum signals received from the plurality of transducer elements for each sub-array according to the time delay values for analog beamforming. Furthermore, the processor 118 may control the ultrasound transmitter/receiver module 110 to perform ADC on the resulting sum signal for each sub-array. In addition, the processor 118 may control the digital beamformer to generate ultrasound data by summing the digital output signals according to the time delay values for digital beamforming.

The processor 118 may control all operations of the probe 20 and operations of components of the probe 20. The processor 118 may execute programs or instructions stored in the memory 111 to perform or control various operations or functions of the probe 20. The processor 118 may also receive a control signal from the input interface 109 of the probe 20 or an external device (e.g., the ultrasound imaging apparatus 40) to control an operation of the probe 20. The processor 118 may also receive a control signal from the input interface 109 or an external device to control an operation of the probe 20. The input interface 109 may receive a user input for controlling the probe 20. For example, the user input may include, but is not limited to, inputs for manipulating buttons, keypads, mice, trackballs, jog switches, or knops, an input for touching a touchpad or a touch screen, a voice input, a motion input, and an input of biometric information (e.g., iris recognition, fingerprint recognition, etc.).

The display 112 may display ultrasound images generated by the probe 20, ultrasound images generated by processing ultrasound data generated by the probe 20, ultrasound images received from the ultrasound imaging apparatus 40, various pieces of information processed by the ultrasound imaging system 100, or the like. In addition, the display 112 may further display status information of the probe 20. The status information of the probe 20 may include at least one of device information of the probe 20, battery status information of the probe 20, frequency band information of the probe 20, output information of the probe 20, information about failures of the probe 20, setting information of the probe 20, or temperature information of the probe 20.

The probe 20 may include one or a plurality of displays 112 depending on its implemented configuration. Furthermore, the display 112 may include a touch panel or a touch screen. The display 112 may also include a flexible display.

The communication module 119 may wirelessly transmit the generated ultrasound data or ultrasound image to the ultrasound imaging apparatus 40 via a wireless network. The communication module 119 may also receive a control signal and data from the ultrasound imaging apparatus 40.

The ultrasound imaging apparatus 40 may receive ultrasound data or an ultrasound image from the probe 20.

In an embodiment, when the probe 20 includes the image processor 130 capable of generating an ultrasound image by using ultrasound data, the probe 20 may transmit ultrasound data or an ultrasound image generated by the image processor 130 to the ultrasound imaging apparatus 40.

In an embodiment, when the probe 20 does not include the image processor 130 capable of generating an ultrasound image by using ultrasound data, the probe 20 may transmit ultrasound data to the ultrasound imaging apparatus 40. Ultrasound data may include ultrasound raw data, and an ultrasound image may mean ultrasound image data.

The ultrasound imaging apparatus 40 may include a processor 120, an image processor 130, a display 140, a memory 150, a communication module 160, and an input interface 170.

The image processor 130 generates or processes an ultrasound image by using ultrasound data received from the probe 20.

The display 140 may display an ultrasound image received from the probe 20, an ultrasound image generated by processing ultrasound data received from the probe 20, various pieces of information processed by the ultrasound imaging system 100, or the like. The ultrasound imaging apparatus 40 may include one or a plurality of displays 140 depending on its implemented configuration. Furthermore, the display 140 may include a touch panel or a touch screen. In addition, the display 140 may include a flexible display.

The processor 120 may control all operations of the ultrasound imaging apparatus 40 and operations of components of the ultrasound imaging apparatus 40. The processor 120 may execute programs or applications stored in the memory 150 to perform or control various operations or functions of the ultrasound imaging apparatus 40. The processor 120 may also control an operation of the ultrasound imaging apparatus 40 by receiving a control signal from the input interface 170 or an external device.

The ultrasound imaging apparatus 40 includes the communication module 160 via which it may be connected to and communicate with external devices (e.g., servers, medical devices, and portable devices such as smartphones, tablet PCs, wearable devices, etc.).

The communication module 160 may include at least one component that enables communication with an external device. The communication module 160 may include, for example, at least one of a short-range communication module, a wired communication module, or a wireless communication module.

The communication module 160 of the ultrasound imaging apparatus 40 may communicate with the communication module 119 of the probe 20 by using a network or a short-range wireless communication method. For example, the communication module 160 of the ultrasound imaging apparatus 40 may communicate with the communication module 119 of the probe 20 by using any one of wireless data communication methods including a wireless local area network (WLAN), Wi-Fi, Bluetooth, ZigBee, Wi-Fi Direct (WFD), Infrared Data Association (IrDA), Bluetooth Low Energy (BLE), near field communication (NFC), wireless broadband Internet (WiBro), World Interoperability for Microwave Access (WiMAX), Shared Wireless Access Protocol (SWAP), Wireless Gigabit Alliance (WiGig), radio frequency (RF) communication, 60 gigahertz (GHz) millimeter wave (mmWave) short-range communication, etc.

To achieve this, the communication module 160 of the ultrasound imaging apparatus 40 and the communication module 119 of the probe 20 may each include at least one of a WLAN communication module, a Wi-Fi communication module, a Bluetooth communication module, a ZigBee communication module, a WFD communication module, an IrDA communication module, a BLE communication module, an NFC communication module, a WiBro) communication module, a WiMAX communication module, a SWAP communication module, a WiGig communication module, an RF communication module, or a 60 GHz mmWave short-range communication module.

In an embodiment, the probe 20 may transmit device information (e.g., identification (ID) information) of the probe 20 to the ultrasound imaging apparatus 40 by using a first communication method (e.g., BLE), and may be paired wirelessly with the ultrasound imaging apparatus 40. Furthermore, the probe 20 may transmit ultrasound data and/or ultrasound images to the paired ultrasound imaging apparatus 40.

The device information of the probe 20 may include various pieces of information related to a serial number, a model name, a battery status, etc. of the probe 20.

The ultrasound imaging apparatus 40 may receive, from the probe 20, the device information (e.g., ID information) of the probe 20 by using the first communication method (e.g., BLE), and may be paired wirelessly with the probe 20. Furthermore, the ultrasound imaging apparatus 40 may transmit an activation signal to the paired probe 20 and receive ultrasound data and/or ultrasound images from the probe 20. In this case, the activation signal may include a signal for controlling an operation of the probe 20.

Furthermore, the probe 20 may transmit ultrasound data and/or ultrasound images to the ultrasound imaging apparatus 40 paired through the first communication method by using a second communication method (e.g., 60 GHz mmWave or Wi-Fi).

Furthermore, the ultrasound imaging apparatus 40 may transmit an activation signal to the paired probe 20 and receive ultrasound data and/or ultrasound images from the probe 20 by using the second communication method (e.g., 60 GHz mmWave or Wi-Fi).

According to an embodiment, the first communication method used to pair the probe 20 and the ultrasound imaging apparatus 40 with each other may have a lower frequency band than the second communication method used by the probe 20 to transmit ultrasound data and/or ultrasound images to the ultrasound imaging apparatus 40.

The display 140 of the ultrasound imaging apparatus 40 may display Uls indicating device information of the probe 20. For example, the display 140 may display Uls indicating ID information of the probe 20, a pairing method indicating a method of pairing the ultrasound imaging apparatus 40 with the probe 20, a status of data communication between the probe 20 and the ultrasound imaging apparatus 40, a method of performing data communication with the ultrasound imaging apparatus 40, a battery status of the probe 20, etc.

When the probe 20 includes the display 112, the display 112 of the probe 20 may display a UI indicating device information of the probe 20. For example, the display 112 may display Uls indicating ID information of the probe 20, a pairing method indicating a method of pairing the probe 20 with the ultrasound imaging apparatus 40, a status of data communication between the probe 20 and the ultrasound imaging apparatus 40, a method of performing data communication with the ultrasound imaging apparatus 40, a battery status of the probe 20, etc.

The communication module 160 may receive a control signal and data from an external device. The processor 120 may control an operation of the ultrasound imaging apparatus 40 in response to the control signal received via the communication module 160.

Furthermore, the processor 120 may transmit a control signal to an external device via the communication module 160 to control the external device in response to the transmitted control signal. The external device may operate in response to a control signal received from the ultrasound imaging apparatus 40, or process data received from the ultrasound imaging apparatus 40.

The external device may receive or download the program or application related to the ultrasound imaging apparatus 40 from the ultrasound imaging apparatus 40, the probe 20, or a server, and install and execute the program or application thereon. The ultrasound imaging apparatus 40, the probe 20, or the server providing a program or application may include a recording medium storing instructions, commands, installation files, executable files, or related data of the program or application. External devices may also be sold with programs or applications installed.

The memory 150 may store various types of data or programs for driving and controlling the ultrasound imaging apparatus 40, input and/or output ultrasound data, ultrasound images, etc.

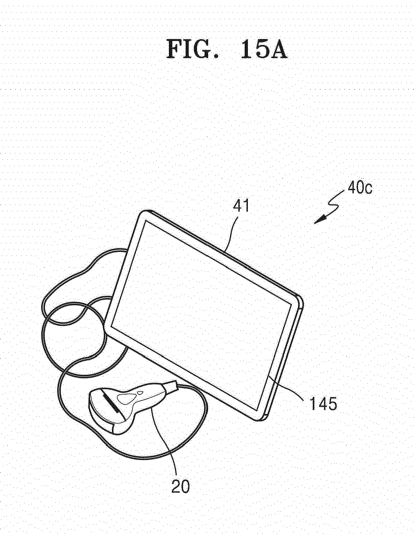

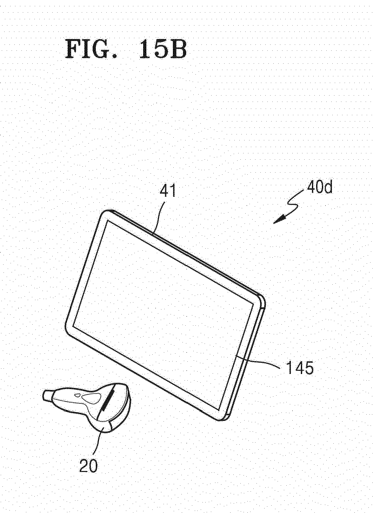

FIGS. 15A and 15B are diagrams illustrating examples of the ultrasound imaging apparatus 40, according to an embodiment.

Referring to FIGS. 15A and 15B, ultrasound imaging apparatuses 40c and 40d may also be implemented as portable ultrasound imaging apparatuses. Examples of the portable ultrasound imaging apparatus may include, but are not limited to, a smartphone, a laptop computer, a PDA, a tablet PC, etc., each of which includes a probe and an application.

The ultrasound imaging apparatus 40c may include a main body 41. Referring to FIG. 15A, the probe 20 may be connected to one side of the main body 41 by wire. To this end, the main body 41 may include a connection terminal to or from which a cable connected to the probe 20 may be attached or detached. The probe 20 may include a cable including a connection terminal connectable to the main body 41.

Referring to FIG. 15B, the probe 20 may be wirelessly connected to the ultrasound imaging apparatus 40d. The main body 41 may include an input/output (I/O) interface such as a touch screen. The I/O interface may display an ultrasound image, various pieces of information processed by the ultrasound imaging apparatus 40d, a graphical user interface (GUI), etc.

The ultrasound imaging apparatus 40d and the probe 20 may establish communication or be paired with each other by using short-range wireless communication. For example, the ultrasound imaging apparatus 40d may communicate with the probe 20 by using Bluetooth, BLE, Wi-Fi, WFD, or the like.

The ultrasound imaging apparatus 40c or 40d may execute a program or application related to the probe 20 to control the probe 20 and output information related to the probe 20. The ultrasound imaging apparatus 40c or 40d may perform operations related to the probe 20 while communicating with a predetermined server. The probe 20 may be registered with the ultrasound imaging apparatus 40c or 40d, or the predetermined server. The ultrasound imaging apparatus 40c or 40d may communicate with the registered probe 20 and perform operations related to the probe 20.

Furthermore, the ultrasound imaging apparatus 40c or 40d may include various types of I/O interfaces such as speakers, light-emitting diodes (LEDs), and vibration devices. For example, the ultrasound imaging apparatus 40c or 40d may output various pieces of information in the form of graphics, sound, or vibrations via the I/O interfaces. In addition, the ultrasound imaging apparatus 40c or 40d may output various notifications or data via the I/O interfaces.

According to an embodiment, the ultrasound imaging apparatus 40a, 40b, 40c, or 40d may process ultrasound images or obtain additional information from the ultrasound images by using an AI model. According to an embodiment, the ultrasound imaging apparatus 40a, 40b, 40c, or 40d may use an AI model to generate ultrasound images or perform processing, such as correction, image enhancement, encoding, or decoding, on the ultrasound images. Furthermore, according to an embodiment, the ultrasound imaging apparatus 40a, 40b, 40c, or 40d may use an AI model to perform processing, such as defining a baseline, obtaining anatomical information, obtaining lesion information, extracting surfaces, defining a boundary, measuring a length, measuring an area, measuring a volume, or generating annotations from an ultrasound image.

An AI model may be provided in the ultrasound imaging apparatus 40a, 40b, 40c, or 40d, or may be provided in a server.

AI models may be implemented using various artificial neural network models or deep neural network (DNN) models. Furthermore, AI models may be trained and generated using various machine learning algorithms or deep learning algorithms. AI models may be implemented using models, such as convolutional neural networks (CNNs), recurrent neural networks (RNNs), generative adversarial networks (GANs), long short-term memory (LSTM), etc.

A machine-readable storage medium may be provided in the form of a non-transitory storage medium. In this regard, the term 'non-transitory storage medium' only means that the storage medium does not include a signal (e.g., an electromagnetic wave) and is a tangible device, and the term does not differentiate between where data is semi-permanently stored in the storage medium and where the data is temporarily stored in the storage medium. For example, the 'non-transitory storage medium' may include a buffer for temporarily storing data.

According to an embodiment, methods according to various embodiments set forth in the present specification may be included in a computer program product when provided. The computer program product may be traded, as a product, between a seller and a buyer. The computer program product may be distributed in the form of a machine-readable storage medium (e.g., compact disc read-only memory (CD-ROM)) or distributed (e.g., downloaded or uploaded) on-line via an application store or directly between two user devices (e.g., smartphones). For online distribution, at least a part of the computer program product (e.g., a downloadable app) may be at least transiently stored or temporally generated in the machine-readable storage medium such as a memory of a server of a manufacturer, a server of an application store, or a relay server.

## Claims

1. An ultrasound imaging apparatus comprising:
a display;
a motion sensor;
an input interface; and
at least one processor configured to
display, via the display, a user interface (Ul) image, corresponding to a measurement parameter, on an ultrasound image,
change, via the motion sensor, a position of the UI image within the ultrasound image according to movement of the ultrasound imaging apparatus,
in response to receiving a user input for setting the measurement parameter via the input interface, determine a value of the measurement parameter based on the position of the UI image within the ultrasound image, and
display a measurement value for an object based on the determined value of the measurement parameter.

2. The ultrasound imaging apparatus of claim 1, wherein the at least one processor is further configured to, in response to a user input for changing at least one of a position or an angle of the ultrasound imaging apparatus, move the ultrasound image in a lateral direction, thereby moving the UI image within the ultrasound image in the lateral direction.

3. The ultrasound imaging apparatus of claim 1, wherein the at least one processor is further configured to, in response to a user input for changing at least one of a position or an angle of the ultrasound imaging apparatus, move the ultrasound image in an axial direction, thereby moving the UI image within the ultrasound image in the axial direction.

4. The ultrasound imaging apparatus of claim 1, wherein the at least one processor is further configured to, in response to a user input for changing at least one of a position or an angle of the ultrasound imaging apparatus, change a size of the UI image within the ultrasound image.

5. The ultrasound imaging apparatus of claim 1, wherein the at least one processor is further configured to, in response to a user input for changing at least one of a position or an angle of the ultrasound imaging apparatus, change an angle of the UI image within the ultrasound image.

6. The ultrasound imaging apparatus of claim 1, wherein the at least one processor is further configured to, in response to a user input for changing at least one of a position or an angle of the ultrasound imaging apparatus, move the ultrasound image up, down, left, or right, thereby moving the position of the UI image within the ultrasound image up, down, left, or right.

7. The ultrasound imaging apparatus of claim 1, wherein
the measurement parameter includes a plurality of measurement parameters having a predetermined setting order, and
the at least one processor is further configured to, in response to receiving a user input for setting a first measurement parameter from among the plurality of measurement parameters, display a UI image corresponding to a second measurement parameter that is a next parameter following the first measurement parameter.

8. The ultrasound imaging apparatus of claim 1, wherein
the at least one processor is further configured to
display the ultrasound image, and
based on receiving a user input for entering a measurement mode, enlarge a region of the displayed ultrasound image, and display the UI image on the enlarged region.

9. The ultrasound imaging apparatus of claim 1, wherein the at least one processor is further configured to display guidance information that guides a direction of movement of the ultrasound imaging apparatus for moving the UI image within the ultrasound image.

10. The ultrasound imaging apparatus of claim 1, wherein
the at least one processor is further configured to,
when a predetermined hardware button is pressed, receive a user input for changing at least one of the position or the angle of the ultrasound imaging apparatus, and
in response to receiving a user input for releasing the pressed predetermined hardware button, determine the position of the UI image within the ultrasound image as a value of the measurement parameter.

11. A method, performed by an ultrasound imaging apparatus, of providing a user interface (Ul), the method comprising:
displaying a UI image, corresponding to a measurement parameter, on an ultrasound image;
changing a position of the UI image within the ultrasound image according to movement of the ultrasound imaging apparatus;
in response to receiving a user input for setting the measurement parameter, determining a value of the measurement parameter based on the position of the UI image within the ultrasound image; and
displaying a measurement value for an object based on the determined value of the measurement parameter.

12. The method of claim 11, wherein the changing of the position of the UI image within the ultrasound image according to the movement of the ultrasound imaging apparatus comprises, in response to a user input for changing at least one of a position or an angle of the ultrasound imaging apparatus, moving the ultrasound image in a lateral direction, thereby moving the UI image within the ultrasound image in the lateral direction.

13. The method of claim 11, wherein the changing of the position of the UI image within the ultrasound image according to the movement of the ultrasound imaging apparatus comprises, in response to a user input for changing at least one of a position or an angle of the ultrasound imaging apparatus, moving the ultrasound image in an axial direction, thereby moving the UI image within the ultrasound image in the axial direction.

14. The method of claim 11, further comprising, in response to a user input for changing at least one of a position or an angle of the ultrasound imaging apparatus, changing a size of the UI image within the ultrasound image.

15. The method of claim 11, further comprising, in response to a user input for changing at least one of a position or an angle of the ultrasound imaging apparatus, changing an angle of the UI image within the ultrasound image.

## Amended claims

### Amended claims in accordance with Rule 137(2) EPC.

1. An ultrasound imaging apparatus (40) comprising:
a display (140);
a motion sensor (190);
a communication module (160);
an input interface (170); and
at least one processor (120) configured to
receive, via the communication module (160), an ultrasound image from a probe (20) imaging an object,
display, via the display (140), a user interface (Ul) image, corresponding to a measurement parameter, on the ultrasound image,
in response to receiving a user input for moving the ultrasound imaging apparatus (40) separately from the probe (20) via the motion sensor (190), change, via the display (140), a position of the UI image on the ultrasound image according to movement of the ultrasound imaging apparatus (40),
in response to receiving a user input for setting the measurement parameter via the input interface, determine a value of the measurement parameter based on the changed position of the UI image on the ultrasound image, and
display, via the display (140) a measurement value for the object based on the determined value of the measurement parameter.

2. The ultrasound imaging apparatus (40) of claim 1, wherein the at least one processor is further configured to, in response to a user input for changing at least one of a position or an angle of the ultrasound imaging apparatus (40), via the display (140), move the ultrasound image in a lateral direction, thereby moving the UI image on the ultrasound image in the lateral direction.

3. The ultrasound imaging apparatus (40) of claim 1, wherein the at least one processor is further configured to, in response to a user input for changing at least one of a position or an angle of the ultrasound imaging apparatus (40), via the display (140), move the ultrasound image in an axial direction, thereby moving the UI image on the ultrasound image in the axial direction.

4. The ultrasound imaging apparatus (40) of claim 1, wherein the at least one processor is further configured to, in response to a user input for changing at least one of a position or an angle of the ultrasound imaging apparatus (40), via the display (140), change a size of the UI image on the ultrasound image.

5. The ultrasound imaging apparatus (40) of claim 1, wherein the at least one processor is further configured to, in response to a user input for changing at least one of a position or an angle of the ultrasound imaging apparatus (40), via the display (140), change an angle of the UI image on the ultrasound image.

6. The ultrasound imaging apparatus (40) of claim 1, wherein the at least one processor is further configured to, in response to a user input for changing at least one of a position or an angle of the ultrasound imaging apparatus (40), via the display (140), move the ultrasound image up, down, left, or right, thereby moving the position of the UI image on the ultrasound image up, down, left, or right.

7. The ultrasound imaging apparatus (40) of claim 1, wherein
the measurement parameter includes a plurality of measurement parameters having a predetermined setting order, and
the at least one processor is further configured to, in response to receiving a user input for setting a first measurement parameter from among the plurality of measurement parameters, display a UI image corresponding to a second measurement parameter that is a next parameter following the first measurement parameter.

8. The ultrasound imaging apparatus (40) of claim 1, wherein
the at least one processor is further configured to
display the ultrasound image, and
based on receiving a user input for entering a measurement mode, enlarge a region of the displayed ultrasound image, and display the UI image on the enlarged region.

9. The ultrasound imaging apparatus (40) of claim 1, wherein the at least one processor is further configured to display guidance information that guides a direction of movement of the ultrasound imaging apparatus (40) for moving the UI image on the ultrasound image.

10. The ultrasound imaging apparatus (40) of claim 1, wherein
the at least one processor is further configured to,
when a predetermined hardware button is pressed, receive a user input for changing at least one of the position or the angle of the ultrasound imaging apparatus (40), and
in response to receiving a user input for releasing the pressed predetermined hardware button, determine the position of the UI image on the ultrasound image as a value of the measurement parameter.

11. A method, performed by an ultrasound imaging apparatus, of providing a user interface (Ul), the method comprising:
receiving an ultrasound image from a probe imaging an object;
displaying a UI image, corresponding to a measurement parameter, on the ultrasound image (S310);
in response to receiving a user input for moving the ultrasound imaging apparatus,
changing a position of the UI image on the ultrasound image according to movement of the ultrasound imaging apparatus (S320);
in response to receiving a user input for setting the measurement parameter, determining a value of the measurement parameter based on the changed position of the UI image on the ultrasound image (S330); and
displaying a measurement value for the object based on the determined value of the measurement parameter (S340).

12. The method of claim 11, wherein the changing of the position of the UI image on the ultrasound image according to the movement of the ultrasound imaging apparatus comprises, in response to a user input for changing at least one of a position or an angle of the ultrasound imaging apparatus, moving the ultrasound image in a lateral direction, thereby moving the UI image on the ultrasound image in the lateral direction.

13. The method of claim 11, wherein the changing of the position of the UI image on the ultrasound image according to the movement of the ultrasound imaging apparatus comprises, in response to a user input for changing at least one of a position or an angle of the ultrasound imaging apparatus, moving the ultrasound image in an axial direction, thereby moving the UI image on the ultrasound image in the axial direction.

14. The method of claim 11, further comprising, in response to a user input for changing at least one of a position or an angle of the ultrasound imaging apparatus, changing a size of the UI image on the ultrasound image.

15. The method of claim 11, further comprising, in response to a user input for changing at least one of a position or an angle of the ultrasound imaging apparatus, changing an angle of the UI image on the ultrasound image.
